# EUROPEAN PATENT SPECIFICATION

(11) **EP 2 175 871 B1**
(45) Date of publication and mention of the grant of the patent: **31.08.2016**
(21) Application number: 08789918.3
(22) Date of filing: 18.07.2008
(51) Int. Cl.: A61K 36/9068, A61K 36/59, A61K 36/81, A61K 36/185, A61K 36/324, A61P 19/02

(54) **A SYNERGISTIC HERBAL COMPOSITION FOR TREATMENT OF RHEUMATIC AND MUSCULO-SKELETAL DISORDERS (RMSDS)**
SYNERGISTISCHE KRÄUTERZUSAMMENSETZUNG ZUR BEHANDLUNG RHEUMATISCHER UND MUSKULOSKELETTALER ERKRANKUNGEN (RMSDS)
COMPOSITION SYNERGIQUE D'ORIGINE VÉGÉTALE POUR LE TRAITEMENT DE TROUBLES RHUMATISMAUX ET MUSCULO-SQUELETTIQUES (RMSDS)

(30) Priority: 18.07.2007 IN DE15242007
(43) Date of publication of application: 21.04.2010
(73) Proprietor: Council of Scientific & Industrial Research, New Delhi 110 001 (IN)
(72) Inventor: PATWARDHAN, Bhushan, Pune 411 007, Maharashtra (IN); CHOPRA, Arvind, Pune 411 001, Maharashtra (IN); NARSIMULU, Gumdal, Hyderabad 500 082, Andhra Pradesh (IN); HANDA, Rohini, Ansari Nargar, New Delhi 110 029 (IN); BICHILE, Lata, S., Mumbai 400 012, Maharashtra (IN); QAZI, Ghulam, Nabi, Jammu-Tawi 18001, Jammu Kashmir (IN); MUJUMDAR, Arvind, Manohar, Pune 411 004, Maharashtra (IN); SUMANTRAN, Venil, N., Pune 411 043, Maharashtra (IN); PUSHPANGADAM, Palpu, Lucknow 226 001, Uttar Pradesh (IN); MEHROTRA, Shanta, Lucknow 226 001, Uttar Pradesh (IN); RAWAT, Ajay, Kumar, Singh, Lucknow 226 001, Uttar Pradesh (IN); KHATOON, Sayyada, Lucknow 226 001, Uttar Pradesh (IN); RASTOGI, Subha, Lucknow 226 001, Uttar Pradesh (IN); RAGHAVAN, Govindarajan, Lucknow 226 001, Uttar Pradesh (IN); RAUT, Ashwinikumar, Mumbai 400 049, Maharashtra (IN)
(74) Representative: Graf von Stosch, Andreas
(86) International application number: PCT/IN2008/000462
(87) International publication number: WO 2009/010992

(56) References cited:
- US-A- 5 494 668
- US-A- 5 683 698
- US-B1- 6 586 015

## Description

### Field of the invention:

The present invention relates to a synergistic herbal composition for use in the treatment of rheumatic and musculo-skeletal disorders (RMSDs) comprising a base formulation (50-60%) and plant parts and/or extracts of the plant (40-50%) selected from the group consisting of *Withania somnifera, Tribulus terrestris, Phyllanthus emblica* and *Boswellia serrata* optionally along with pharmaceutically acceptable excipients, wherein the base formulation essentially consists of *Zingiber officinale* and *Tinospora cordifolia* in a ratio in the range of 10:90 - 20:80.

The present invention further relates to a process for the preparation of the synergistic herbal composition useful for treatment of rheumatic and musculo-skeletal disorders (RMSDs).

### Background and prior art of the invention:

Rheumatic and Musculoskeletal Disorders (RMSD) including osteoarthritis and rheumatoid arthritis predominantly contribute to the morbidity across the globe, in terms of impaired quality of life (QOL). The term Arthritis" is often used as a more general term to refer to the more than 100 rheumatic diseases that may not only affect the joints but can also cause pain, swelling, and stiffness in other supporting structures of the body such as muscles, tendons and ligaments. The two most common forms of arthritis are osteoarthritis and rheumatoid arthritis.

Osteoarthritis is a worldwide heterogeneous group of conditions that leads to joint symptoms, which are associated with defective integrity of articular cartilage, in addition to related changes in the underlying bone at the joint margins. The etiology of osteoarthritis is multifactorial, with the end result being mechanical joint failure and varying degrees of loss of joint function. Essential inflammatory cytokines, such as IL-1beta and TNF-alpha, are involved initiating a vicious cycle of catabolic and degradative events in cartilage, mediated by metalloproteinases, which degrade cartilage extra-cellular matrix. Nitric oxide, which exerts pro-inflammatory effects, is released during inflammation. (Chikanza, I. et. al., Expert Opin Investig Drugs, 2000, 9(7): 1499-1510).

Rheumatoid arthritis is a chronic syndrome characterized by non-specific usually symmetric inflammation of the peripheral joints, potentially resulting in progressive destruction of articular and periarticular structures leading to deformity. There is a wide spectrum of disease severity but many patients run a course of intermittent relapses and remissions with an overall pattern of slowly progressive joint destruction and deformity. Persistent inflammation produces symptoms and damages tissue causing loss of cartilage, erosion of bone matter and subluxation of joint. This results in a high degree of morbidity resulting in disturbed daily life of the patient. The etiology of this debilitating disease is unknown, however it is considered to be immuno-pathalogical in origin. It is believed to be the result of the presence of a relevant antigen to an immuno-genetically susceptible host. Presence of rheumatoid factor in blood, increase in the erythrocyte sedimentation rate and induced radiological changes are used for classification and correct diagnosis.

RMSDs including Rheumatoid Arthritis, Osteoarthritis, dysplasia, lupus, bursitis, and gout, are all characterized by inflammation and pain in bones, joints, muscles, and related connective tissues. Most of the forms are chronic and degenerative leading to loss of effective use of affected joints. Thus the goal of therapeutic methods for treating RMSDs is the relief of pain and discomfort and the restoration of use of inflamed joints. The novel strategies under consideration for the treatment of RMSDs can be divided into five main areas. These are COX-2 inhibitors, nitric oxide synthesis inhibitors and anti-oxidants, chondrocyte and bone growth promoters, metalloproteinase and cytokine inhibitors and gene therapy.

Present treatment of Arthritis includes first line drugs for control of pain and inflammation classified as non-steroidal, anti-inflammatory drugs (NSAIDS) such as celecoxib, ibuprofen, aspirin etc. Secondary treatment include corticosteroids, slow acting anti-rheumatic drugs (SAARDS) or disease modifying (DM) drugs include penicillinamine like drugs such as cyclophosphamide, methotrexate, gold salts, azothioprine, levamisole and the like. All these drugs have severe side effects and most of them are cytotoxic. For example, non-steroidal anti-inflammatory drugs (NSAIDS) can have a variety of toxic side effects such as gastric erosion and adverse effects on kidneys and liver, and may inadequately regulate the cellular immune functions and secretions of various cytokines. Out of the aforesaid drugs, only levamisole can be categorized as immunostimulatory and has been reported to reduce rheumatoid factor titers.

Drugs targeting pain and inflammation are most often prescribed for musculoskeletal diseases. However, there are few drugs, which target cartilage repair. Glucosamine and Chondroitin sulphate have been prescribed for the past few years. The therapeutic effects of these dietary supplements remain to be proven. One study suggests aberrant immune responses to glycosaminoglycans (GAGs), a major component of joint cartilage, joint fluid, and other soft connective tissue, causes rheumatoid arthritis. The study shows that injection of GAGs such as hyaluronic acid, heparin, and chondroitin sulfates A, B, and C induce arthritis, tendosynovitis, dermatitis, and other pathological conditions in mice. (Wang, J.Y. et. al., Proc Natl Acad Sci, 2002, 99 (22): 14362-14367).

The use of biological agents such as TNF-alpha antagonists etanercept, infliximab, adalimumab and selective interleukin-1 blocker, anakinra has been recently approved for the treatment of rheumatoid arthritis.

Botanicals are also widely being used to treat musculoskeletal diseases. Some patented formulations composed of botanicals targeting musculoskeletal diseases are disclosed.

U.S. Patent No. 5,494,668 (Patwardhan) discloses a method of treating degenerative musculoskeletal diseases such as rheumatoid arthritis and osteoarthritis in an animal, typically a human, involving administering to the animal, typically enterally, in a convenient dosage form, a therapeutically effective amount of the beneficiated extracts of the plants Ashwagandha, Sallai Guggul, Turmeric, and Ginger, in a predetermined proportion relative to each other with or without other biologically active inorganic ingredients.

U.S. Patent No. 5, 683,698 (Chavali et. al.) discloses an herbal formulation and its use for reducing/ alleviating symptoms associated with rheumatoid arthritis, osteoarthritis, and reactive arthritis and for reducing the production of pro-inflammatory cytokines. The formulation contains an herbal extract from the roots, rhizomes and/or vegetation of six herbal plant varieties, specifically, the species of Alpinia, Smilax, Tinospora, Tribulus, Withania, and Zingiber. The patent further discloses foods, beverages and medicaments in the form of capsules, tablets, liquids, and the like, containing the herbal formulation.

U.S. Patent No. 5,916,565 (Rose et. al.) discloses an orally administered composition for prophylaxis and therapy of joint and connective tissue disorders in vertebrates, wherein the composition contains metabolic precursors, herbal phytochemicals, and palatability agents. Suitable herbal phytochemicals are said to include cayenne, ginger, turmeric, yucca, Devil's claw, nettle leaf, Black Cohosh, alfalfa and celery seeds.

U.S. Patent No. 5,888,514 (Weisman) discloses a composition for treating bone or joint inflammation in mammals, the composition contains a systemically absorbable cartilage and an aminosaccharide and may optinally contain, among other ingredients, one or more extracts of an herb of the genus Withania, of the bark of an herb of the genus Salix, or of a root of an herb of the genus Panax.

U.S. Patent No. 5,908,628 (Hou) discloses compositions for treating osteoarhritis and rheumatoid arthritis, containing talc, silkworm excrement, and ingredients of plants of species of the genera stephania, oix, Pinellia, Prunus, Phellodendron, Sophora, Tetrapanax, Stemona, Glycerrhiza, tripterygium, Forsythia, and Siegesbeckia.

U.S. Patent No. 5,788,971 (Togasaki) discloses an active oxygen free radical scavenging agent composed of green tea leaf extract containing epigallo catechin gallate and sunflower seed extract containing chlorogenic acid.

U.S. Patent No. 5,910,307 (Kwak, et.al.) discloses a combined medicinal plant composition for alleviating acute/chronic inflammation, composed of Clematis Radix, Trichosanthes root, and Prunella herba (which contains oleanolic acid ursolic acid) in a certain ratio. The composition is also useful for inhibiting platelet/whole blood aggregation and inflammation-inducing enzymes (5-lipoxygenase, cyclooxygenase-1 and cyclooxygenase-2) and for scavenging toxic active oxygen species.

U.S. Patent No. 6,024,960 (Kharazmi et. al.) discloses a formulation for the treatment of the symptoms associated with inflammation including arthritis and for the prophylaxis of arthritic disease and inflammation. It further describes a formulation derived from rose-hip for the treatment of symptoms associated with inflammation, including arthritis.

U.S. Patent No. 6,187,314 (Xie et al.) discloses different compositions extracted from Gingko biloba leaves. Said compositions comprise new active components. It also describes a method of preparation of the compositions and individual components of said compositions. Uses of the compositions for arthritis and various other conditions are provided.

U.S. Patent No. 6,193,977 (Han et. al.) discloses a pharmaceutical composition comprising an extract of a mixture of Anemarrhena Rhizoma, a member of family Liliaceae and, Phellodendron bark, a member of family Rutaceae that produces nalgesic and anti-inflammatory effects, and its preparing method. The composition is applicable to act on inflammation and pain, for example, chronic gastritis, arthralgia, benign prostrate hyperplasia, chronic and recurrent cystitis, cervical disc, degenerative joint arthritis, rheumatoid arthritis, tennis elbow, osteoporotic pain, migraine, diabetic neuropathy pain, right flank pain etc.

U.S. Patent No. 6,224,871 (Hastings, et. al.) discloses a dietary supplement for nutritionally promoting healthy joint function in human subjects. The supplement includes as a major ingredient a protein derived from enzymatic hydrolysis of collagen in combination with lesser proportions of glucosamine sulfate, gingko biloba, borage oil powder, turmeric, Boswelia *serrata*, ashwagandha, *Piper nigrum* extract and a herbal blend.

U.S. Patent No. 6,264,995 (Newmark, et. al.) discloses a herbal composition capable of reducing inflammation in bones and joints. It also describes methods of using such herbal composition to reduce inflammation. More, particularly, the herbal composition contains therapeutically effective amounts of the supercritical extracts of ginger, rosemary and oregano, and therapeutically effective amounts of extracts of holy basil, turmeric, green tea, huzhang, Chinese goldthread, barberry, rosemary and Scutellariae baicalensis.

U.S. Patent No.6,274,176 (Tomer, et al) discloses an edible composition for use as anti-inflammatory agent for alleviation of arthritis and gout in mammals. The edible composition is a mixture of at least three, preferably at least seven, herbs collected from the group consisting of *Tanacetum parthenium, Zingiber officinale, Curcuma longa, Coriandrum sativum, Centella asiatica, Oenothera biennis, Valeriana officinalis, Tabebuia impetiginosa, Thymus vulgaris and Sambucus nigra.*

U.S. Patent No. 6,541,045 (Charters et. al.) discloses a herbal composition for combating inflammation, comprising therapeutically effective amounts of Japanese knotweed, Devil's claw, grapeskin, and syzygium. Also provided is an herbal composition for soothing muscles and joints, comprising therapeutically effective amounts of Japanese knotweed, N-acetyl D-glucosamine, chondroitin sulfate, D-glucosamine hydrochloride, methylsulfonylmethane, grapeskin, syzygium, and Devil's claws. Methods of using the formulations are also provided.

U.S. Patent No. 6,576,271 (Nair et. al.) discloses a method for inhibiting cyclooxygenase enzymes and inflammation in a mammal using a cherry or cherry anthocyanins, bioflavonoids and phenolics. In particular a mixture including the anthocyanins, the bioflavonoids and the phenolics is described for this use. The method and compositions are useful for the treatment of arthritis.

JP 11-071290 (Toshiyuki et. al.) discloses a hyaluronidase inhibitor improved in inhibitory effects and high in safety, useful as a therapeutic agent for rheumatism and deformant arthritis, containing an extract of Phyllanthus emblucus as an active ingredient.

### Objects of the invention:

The main object of the present invention is to provide a synergistic herbal composition as defined in claim 1 for use in the treatment of rheumatic and musculo-skeletal disorders (RMSDs).

Another object of the present invention is to provide a process as defined in claim 12 for the preparation of the synergistic herbal composition useful for treatment of rheumatic and musculo-skeletal disorders (RMSDs).

Accordingly, the present invention provides a synergistic herbal composition treatment of rheumatic and musculo-skeletal disorders (RMSDs) comprising a base formulation (50-60%) and plant parts and/or extracts of the plant (40-50%) selected from the group consisting of Withania somnifera, Tribulus terrestris, Phyllanthus emblica and Boswellia serrata and any combination thereof optionally along with pharmaceutically acceptable excipients, wherein the base formulation essentially consists of Zingiber officinale and Tinospora cordifolia in a ratio in the range of 10:90 - 20:80.

In another embodiment of the present invention, the said formulation preferably comprising Zingiber officinale, Tinospora cordifolia, Withania somnifera and Tribulus terrestris in a ratio in the range of 20:40:20:20-30:20:25:25 forming formulation B having peripheral analgesic activity and acute inflammatory activity.

In still another embodiment of the present invention, the said formulation further preferably comprising *Zingiber officinale, Tinospora cordifolia, Phyllanthus emblica* and *Boswellia serrata* in a ratio in the range of 1:4:8:5 - 3:5:12:7 forming formulation (C+G).

In yet another embodiment of the present invention, the said formulation is capable of causing simultaneous long term decrease in glycosaminoglycan (GAG) release and aggrecan by cartilage explants resulting in reduction of pain, inflammation, stiffness and low degeneration of bones, joints, muscles and other connective tissues.

In still another embodiment of the present invention, the said formulation is safe for a period in the range of 14 days to 24 weeks time period.

In yet another embodiment of the present invention, the said formulation is non toxic at least up to a single dose of 2000mg/Kg body weight of the subject in need.

In still another embodiment of the present invention, the therapeutically effective dose of the said formulation is in the range of 20-300mg/Kg body weight of the subject in need.

Further in another embodiment of the present invention, the said formulation is capable of being used as an analgesic in an effective dose in the range of 10-100mg/Kg of body weight of the subject in need.

In still another embodiment of the present invention, the said formulation is capable of being used as an anti-inflammatory agent in an effective dose in the range of 50-400mg/Kg of body weight of the subject in need.

In yet another embodiment of the present invention, the said formulation is having peripheral analgesic activity and acute inflammatory activity at an effective dose in the range of 25-400mg/Kg of body weight of the subject in need.

In still another embodiment of the present invention, the said formulation is capable of being used as a sub-acute anti-inflammatory agent in an effective dose in the range of 20-300 mg/Kg of body weight of the subject in need.

In yet another embodiment of the present invention, the said formulation is capable of protecting against Nitric oxide damage in human osteo-arthritic chondrocytes.

In yet another embodiment of the present invention, the said formulation is capable of protecting against Nitric oxide damage in human osteo-arthritic chondrocytes up to 30+11%.

In yet another embodiment of the present invention, the said formulation B is capable of inhibiting hyaluronidase enzyme activity up to 20.8+7%. (Kindly provide experiment/ data/ example to support the statement) Refer data in cell line studies.

In still another embodiment of the invention the said formulation is capable of protecting against peroxide damage.

In still another embodiment of the invention the said formulation (C+ G) is inhibiting hyaluronidase enzyme activity up to 33.58 ±10.6%.

Further in another embodiment of the invention the formulation is useful for Symptomatic treatment of Osteoarthritis(OA) up to 12 months for therapy.

Further in another embodiment of the present invention, the formulation (C+G) is at least up to 25-40% more effective than glucosamine for reducing the glycosaminoglycan (GAG) and aggrecan release.

In still another embodiment of the present invention, the said formulation is in the form selected from the group comprising powder, capsule, tablet, liquid, paste.

Further in another embodiment of the present invention, a process for the preparation of a synergistic herbal composition according to claim 1, comprising the following steps of:
a. grinding the plant materials to obtain particle size ranging between 2 - 5mm for obtaining powdered plant material;
b. optionally packing the coarse powder in an extractor followed by water-extraction method for obtaining an plant extract;
c. charging water into the powder as obtained from step (a) followed by heating at a temperature range of 90 - 95 degree C for a period of at least up to 3 hours;
d. concentrating the thick paste as obtained from step (c) under vacuum followed by drying using a drier then milling and sieving thereof to obtain the desired formulation of dried plant powder or plant extract.

In yet another embodiment of the present invention, the plant materials are selected from the group comprising a base formulation of *Zingiber officinale* and *Tinospora cordifolia,* and plant parts and/or extract of plants selected from the group consisting of *Withania somnifera, Tribulus terrestris, Phyllanthus emblica, Boswellia serrata.*

In still another embodiment of the present invention, the formulation B comprising rhizome of *Zingiber officinale,* stem of *Tinospora cordifolia,* roots of *Withania somnifera* and fruits of *Tribulus terrestris.*

In yet another embodiment of the present invention, the plant part used is selected from the group comprising stem, bark, root, flower bud and fruit.

In still another embodiment of the present invention, the *Withania somnifera* used being in the range of 25-30% by weight of the said herbal formulation.

In still another embodiment of the present invention, the *Tribulus terrestris* used being in the range of 10-20% by weight of the said herbal formulation.

In yet another embodiment of the present invention, the *Phyllanthus emblica* used being in the range of 10-30% by weight of the said herbal formulation.

Further in an embodiment of the present invention, the *Boswellia serrata* used being in the range of 20-35mg/kg body weight by weight of the said herbal formulation.

In still another embodiment of the present invention, the excipient used is preferably starch.

In yet another embodiment of the present invention, the sieving is performed preferably in a sifter using 30-50µm mesh size.

Further the present application also discloses a non-claimed of protecting against rheumatic and musculoskeletal disorders (RMSDs), wherein the said method comprising the steps of administering therapeutically effective amount of a synergistic herbal formulation to a subject in need.

In yet another embodiment of the present invention, the subject is mammal including human.

In still another embodiment of the present invention, the route of administering is selected from the group comprising enteral, oral, intra-muscular, intra-peritoneal and intra-venous.

### Brief description of the drawings:

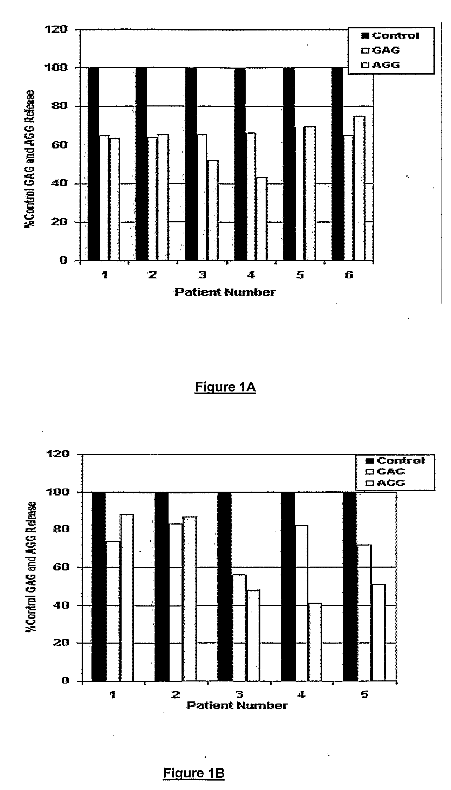

Figure 1A illustrates the effects of Formulation C+G on OA cartilage damage at time point 3. Cartilage explants from six patients treated with Formulation C+G, released 65.94 + 12.24% of levels of Glycosaminoglycan (GAG, white bars), and 62.13 + 22.25% of levels of aggrecan (AGG, gray bars), respectively; compared to controls (set at 100%, black bars). Relative to control explants from these six patients, Formulation C+G decreased GAGs and_Aggrecan release from these explants by a significant, mean, 34% and 38% respectively.
Figure 1B illustrates the effects of Glucosamine on OA cartilage damage at time point 3. Cartilage explants from five of the six patients were treated with Glucosamine. These explants released 73.72 + 9.69% of GAG (white bars), and 66.13 + 21.81 % of aggrecan (AGG, gray bars), respectively; compared to controls (set at 100%, black bars). Relative to control explants from these 5 patients, Glucosamine decreased GAGs and Aggrecan release from explants by a significant, mean, 26% and 34% respectively.

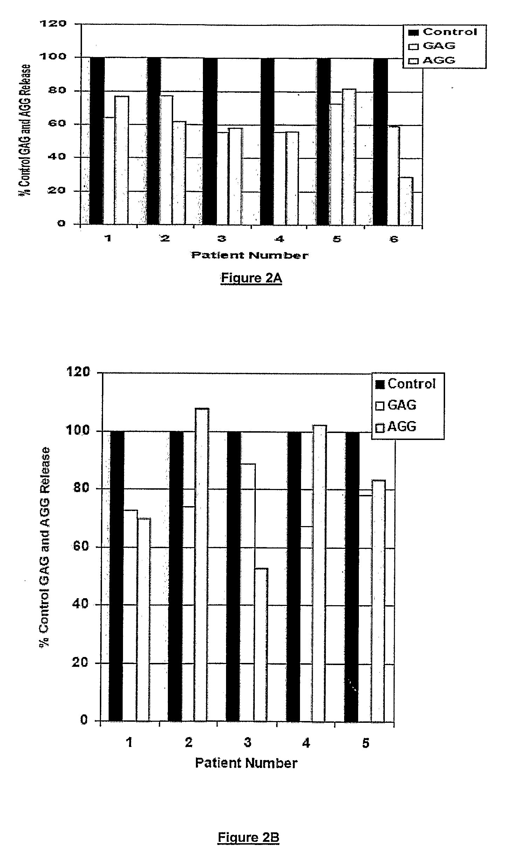

Figure 2A illustrates the effects of Formulation C+G on OA cartilage damage at time point 4. At time point 4, cartilage explants from six patients treated with Formulation (C+G) released 63.62+18.25 % of GAG (white bars), and 60.77+23.96 % of aggrecan (AGG, gray bars), respectively; relative to controls (100%, black bars). Relative to control explants from these 6 patients, Formulation C+G decreased GAGs and Aggrecan release from explants by a significant, mean, 34% and 40% respectively.
Figure 2B illustrates the effects of Glucosamine on OA cartilage damage at time point 4. At time point 4, Glucosamine treated explants released 81.38+14.35% of levels of GAG (white bars), and 86.07+21.71% of levels of aggrecan (AGG, gray bars), respectively; compared to controls (set at 100%, black bars). Glucosamine caused a statistically significant, mean, 20% decrease in levels of GAG released by explants at this time point from these OA patients. Aggrecan release by cartilage explants from these 5 patients was not significantly decreased by Glucosamine at this time point.
   Table 1 shows the summary of Chondroprotective activity of formulations
   Table 2 shows the comparison of Anti-arthritic activities of the four formulations
   Table 3 shows the baseline mean and mean of percent change from baseline over 16 weeks
   Table 4 shows the knee status change on completion - a RIDIT (relative to identified distribution) analysis
   Table 5 shows the Daily consumption Paracetamol (n=202)
   Table 6 shows Baseline mean & mean percent change over 6 weeks
   Table 7shows Mean change between baseline and completion

### Detailed description of the invention:

### Ayurvedic Rationale

Osteoarthritis is known as Sandhigatavata in Ayurvedic terminology. It is a typical Vatavyadhi characterized by vitiation of Vata and degeneration of dhatus. Pain, swelling, crepitus, stiffness and loss of movements of the joint are the major symptoms of the disease. An exhaustive literature search considering all these factors formed the basis for development of the formulations. The base formulation (shunthi +guduchi) is mainly indicated in case of pain. Both the drugs are Vatahara and the synergistic action of this combination is reported (Sharma P.V. (Ed.), Dhanwantari nighantu, Chaukhambha Orientalia, Varanasi, Guduchyadi Varga, 1 st edition, 1982). It is included in some Ayurvedic prescriptions e.g. Rasnadi kwath (Parashuram Shastri (Ed.), Sharangadhara Samhita, Chaukhambha Orientalia, Varanasi, Madhyamkhanda 2:85-87) which is a broad-spectrum effective drug for Arthritis. This combination is expected to act as a pain-relieving drug.

Addition of Ashwagandha and Gokshur to base formulation is expected to act more effectively for pain, degeneration and stiffness.

Guduchi + Amalaki is a part of *Asthimajja Pachaka.* This combination is prescribed for bone and joint related ailments in classical Ayurvedic practice. Both the drugs are Tridoshahara and Rasayana.

Addition of guggul further augments activity due to its Vaatahara and Rasayana property, target action on joints and bones and compatibility with Shunthi and Guduchi combination.

The present invention provides a synergistic herbal composition for use in the treatment of rheumatic and musculo-skeletal disorders (RMSDs) comprising a base formulation (50-60%) and plant parts and/or extracts of the plant (40-50%) selected from the group consisting of Withania somnifera, Tribulus terrestris, Phyllanthus emblica and Boswellia serrata and any combination thereof optionally along with pharmaceutically acceptable excipients, wherein the base formulation essentially consists of Zingiber officinale and Tinospora cordifolia in a ratio in the range of 10:90 - 20:80.

In any disease where multiple therapeutic interventions are necessary, the poly herbal formulation is designed to cover the array maximally. In many case, it happens that the single ingredient may be much more powerful but when combined in formulation it may or may not show similar activity. Still this is preferred because although it may not add up to that particular activity at the desired level, it does add to the spectrum of therapeutic activity. Here in one formulation we are combining various activities such as analgesic, anti inflammatory, anti oxidant, cytoprotective, cartilage protective, etc. Therefore, the putative activity of the composition can be in the range of the activity inherent to an individual component.

In another embodiment of the present invention, the said formulation preferably comprising Zingiber officinale, Tinospora cordifolia, Withania somnifera and Tribulus terrestris in a ratio in the range of 20:40:20:20-30:20:25:25 forming formulation B having peripheral analgesic activity and acute inflammatory activity.

In still another embodiment of the present invention, the said formulation further preferably comprising *Zingiber officinale, Tinospora cordifolia, Phyllanthus emblica* and *Boswellia serrata* in a ratio in the range of 1:4:8:5 - 3:5:12:7 forming formulation (C+G).

In yet another embodiment of the present invention, the said formulation is capable of causing simultaneous long term decrease in glycosaminoglycan (GAG) release and aggrecan by cartilage explants resulting in reduction of pain, inflammation, stiffness and low degeneration of bones, joints, muscles and other connective tissues.

In still another embodiment of the present invention, the said formulation is safe for a period in the range of 14 days to 24 weeks time period.

In yet another embodiment of the present invention, the said formulation is non toxic at least up to a single dose of 2000mg/Kg body weight of the subject in need.

In still another embodiment of the present invention, the therapeutically effective dose of the said formulation is in the range of 20-300mg/Kg body weight of the subject in need.

Further in another embodiment of the present invention, the said formulation is capable of being used as an analgesic in an effective dose in the range of 10-100mg/Kg of body weight of the subject in need.

In still another embodiment of the present invention, the said formulation is capable of being used as an anti-inflammatory agent in an effective dose in the range of 50-200mg/Kg of body weight of the subject in need.

In yet another embodiment of the present invention, the said formulation is having peripheral analgesic activity and acute inflammatory activity at an effective dose in the range of 25-400mg/Kg of body weight of the subject in need.

In still another embodiment of the present invention, the said formulation is capable of being used as a sub-acute anti-inflammatory agent in an effective dose in the range of 20-300 mg/Kg of body weight of the subject in need.

In yet another embodiment of the present invention, the said formulation is capable of protecting against Nitric oxide damage in human osteo-arthritic chondrocytes.

In yet another embodiment of the present invention, the said formulation is capable of protecting against Nitric oxide damage in human osteo-arthritic chondrocytes up to 30+11%.

In yet another embodiment of the present invention, the said formulation B is capable of inhibiting hyaluronidase enzyme activity up to 20.8+7%. (Kindly provide experiment/ data/ example to support the statement) Refer data in cell line studies.

In still another embodiment of the invention the said formulation is capable of protecting against peroxide damage.

In still another embodiment of the invention the said formulation (C+ G) is inhibiting hyaluronidase enzyme activity up to 33.58 ±10.6%.

Further in another embodiment of the invention the formulation is useful for Symptomatic treatment of Osteoarthritis (OA) up to 12 months for therapy.

Further in another embodiment of the present invention, the formulation (C+G) is at least up to 25-40% more effective than glucosamine for reducing the glycosaminoglycan (GAG) and aggrecan release.

In still another embodiment of the present invention, the said formulation is in the form selected from the group comprising powder, capsule, tablet, liquid, paste.

Further in another embodiment of the present invention, a process for the preparation of a synergistic herbal composition according to claim 1 comprising the following steps of:
a. grinding the plant materials to obtain particle size ranging between 2 - 5mm for obtaining powdered plant material;
b. optionally packing the coarse powder in an extractor followed by water-extraction method for obtaining an plant extract;
c. charging water into the powder as obtained from step (a) followed by heating at a temperature range of 90 - 95 degree C for a period of at least up to 3 hours;
d. concentrating the thick paste as obtained from step (c) under vacuum followed by drying using a drier then milling and sieving thereof to obtain the desired formulation of dried plant powder or plant extract.

In yet another embodiment of the present invention, the plant materials are selected from the group comprising a base formulation of *Zingiber officinale* and *Tinospora cordifolia,* and plant parts and/or extract of plants selected from the group consisting of *Withania somnifera, Tribulus terrestris, Phyllanrhus emblica, Boswellia serrata.*

In still another embodiment of the present invention, the formulation B comprising rhizome of *Zingiber officinale,* stem of *Tinospora cordifolia,* roots of *Withania somnifera* and fruits of *Tribulus terrestris.*

In yet another embodiment of the present invention, the plant part used is selected from the group comprising stem, bark, root, flower bud and fruit.

In still another embodiment of the present invention, the *Withania somnifera* used being in the range of 25-30% by weight of the said herbal formulation.

In still another embodiment of the present invention, the *Tribulus terrestris* used being in the range of 10-20% by weight of the said herbal formulation.

In yet another embodiment of the present invention, the *Phyllanthus emblica* used being in the range of 10-30% by weight of the said herbal formulation.

Further in an embodiment of the present invention, the *Boswellia serrata* used being in the range of 20-35mg/kg body weight by weight of the said herbal formulation.

In still another embodiment of the present invention, the excipient used is preferably starch.

In yet another embodiment of the present invention, the sieving is performed preferably in a sifter using 30-50µm mesh size.

Further the present application also discloses, a non-claimed method of protecting against rheumatic and musculoskeletal disorders (RMSDs), wherein the said method comprising the steps of administering therapeutically effective amount of a synergistic herbal formulation to a subject in need.

In yet another embodiment of the present invention, the subject is mammal including human.

In still another embodiment of the present invention, the route of administering is selected from the group comprising enteral, oral, intra-muscular, intra-peritoneal and intra-venous.

### Ginger (Zingiber officinale)

Ginger inhibits both COX-2 and 5-LOX and further functions as an antioxidant [6]-gingerol is a potent inhibitor of NO synthesis and also an effective protector against peroxynitrite-mediated damage. In the present invention crude ginger powder is used.

Ginger contains 1-4% essential oil (oleoresin). This essential oil contains mixture of various terpenes as well as some other non-terpenoid compounds. Due to large battery of compounds belonging to various chemical classes, it is likely that crude ginger powder intake brings about amelioration of symptoms by interfering with the production and release of products of lipid membranes (eicosanoids, reactive oxygen), peptides and proteins (lysosomal enzymes, growth factors, lymphokines, bradykinin), amino acids (histamin, serotonin) etc. (Kiuchi F.et. al, Chemical and Pharmaceutical Bulletin, 1982, 30:747-754). The modulatory effects of volatile oil of ginger were studied on the cellular immune response in vitro and in vivo in mice. The results suggest that it influences both cell-mediated immune response and nonspecific proliferation of T lymphocyte, and may exert beneficial effects in a number of clinical conditions, such as chronic inflammation and autoimmune diseases (Zhou, H. L. et al., J Ethnopharmacol, 2006, 105(1-2):301-5)

Ginger gives relief from muscular discomfort and pain. In a multicentric, randomnized controlled trial, a highly purified and standardized ginger extract showed statistically significant effect on reducing symptoms of OA of the knee. (Altman, R.D. et. al., Arthritis Rheum, 2001, 44(11): 2531-8). It inhibits prostaglandin and leukotriene biosynthesis and histamine release and thus acts as an anti-inflammatory agent. (Thomson, M. et. al., Prostaglandins Leukot Essent Fatty Acids, 2002, 67(6): 475-8; Surh, Y.J. et. al., Food Chem Toxicol, 2002, 40(8): 1091-7; Shen, C.J. et. al., J Med Food, 2005, 8(2):149-53; Lantz, R.C. et. al., Phytomed, 2006; Ojewole, J.A., Phytother Res, 2006).Ginger root has been used for thousands of years to treat inflammatory diseases, including osteoarthritis (Grzanna, R. et. al., J Med Food, 2005, 8(2):125-32).

Ginger extract was compared to placebo and Ibuprofen in patients with osteoarthritis of the hip or knee in a controlled, double blind, double dummy, cross-over study with a wash-out period of one week followed by three treatment periods in a randomized sequence, each of three weeks duration. Acetaminophen was used as rescue medication throughout the study. A ranking of efficacy of the three treatment periods: lbuprofen>ginger extract>placebo was found for visual analogue scale of pain (Friedman test: 24.65, P< 0.00001) and the Lequesne-index (Friedman test: 20.76, P< 0.00005). In the cross-over study, no significant difference between placebo and ginger extract could be demonstrated (Siegel-Castellan test), while explorative tests of differences in the first treatment period showed a better effect of both. Ibuprofen and ginger extract than placebo (Chi-square, P< 0.05). There were no serious adverse events reported during the periods with activemedications. (Blidda, H. et. al., Osteoarthritis Cartilage, 2000, 8(1):9-12).Effect of different concentrations (0-2,000 microg/mL) of ginger root extract (GRE) on the viability and the production of nitric oxide (NO) and prostaglandin E(2) (PGE(2)) by sow osteoarthrotic cartilage explants has been investigated. Increasing GRE concentration (1-100 microg/mL) reduced (P <.05) NO and PGE (2) production suggesting an important role for GRE as an anti-arthritic agent in osteoarthrosis in sow. (Shen, C.L. et. al., J Med Food,2003, 6(4):323-8).

It is a dual-inhibitor of the lipoxygenase and cyclooxygenase system.(Srivastava, et. al, Medical Hypotheses, 1992,39:342-48). Melatonin, a constituent of ginger, has been found to exert potent anti-inflammatory effects via COX-2 inhibition. (Cuzzocrea, S. et. al, J. Pineal Res., 1999, 27(1):9-14; Hattori, A. et. al., Biochem. Mol Biol Int, 1995, 35(3):627-34; Srivastava, K.C. et al., Biomed Biochim Acta, 1984, 43(8-9): S335-46; Katiyar, S. et. al., Cancer Res, 1996, 56(5):1023-30). The ability of 20 pungent constituents of ginger and related substances to inhibit arachidonic acid (AA) induced platelet activation in human whole blood was studied. [8]-Gingerol, [8]-shogaol, [8]-paradol and gingerol analogues (1 and 5) exhibited anti-platelet activities with IC(50) values ranging from 3 to 7 microM, whilst under similar conditions the IC(50) value for aspirin was 20+/-11 microM. The findings show that gingerol compounds and their derivatives are more potent anti-platelet agents than aspirin under the conditions described in this study. [8]-Paradol, a natural constituent of ginger, was found to be the most potent COX-1 inhibitor and anti platelet aggregation agent. The mechanism underlying AA-induced platelet aggregation inhibition may be related to attenuation of COX-1/Tx synthase enzymatic activity. (Nurtjahja-Tjendraputra, E. et. al., Thromb Res, 2003, 111(4-5): 259-65). Another constituent of ginger, eugenol, has also been found to be a 5-lipoxygenase inhibitor and to possess potent anti-inflammatory and /or anti-rheumatic properties.(Sharma, J.N. et. al., Pharmacology, 1994, 49(5):314-8). The efects of the crude hydralcoholic extract of ginger rhizomes have been investigated on the classical models of rat paw and skin edema. The carrageenan-, compound 48/80- or serotonin-induced rat paw edema was inhibited significantly by the intraperitoneal administration of alcoholic ginger extract. The antiedematogenic activity seems to be related, at least partially, to an antagonism of the serotonin receptor. (Penna, S.C. et. al., Phytomedicine,2003,10(5):381-5)

Recent reports suggest ginger might be a potent and novel therapeutic agent for scavenging of NO and the regulation of pathological conditions caused by excessive generation of NO and its oxidation product, peroxynitrite. (Baliga, M.S. et. al., Nahrung, 2003,47 (4):261-4). Another study indicates that [6]-gingerol is a potent inhibitor of NO synthesis and also an effective protector against peroxynitrite-mediated damage. (Ippoushi, K. et. al., Life Sci, 2003, 73(26): 3427-37).

Used alone fresh ginger is required to be used in substantially high doses (50gm daily), which is not only inconvenient but can act as an irritant to the gastric mucosa. In dry form for any significant results 7-10gm of dry ginger powder has to be taken daily. These therapeutic doses of ginger are extremely inconvenient for the patient and affect compliance on a daily basis. (Patwardhan, U.S. Pat. No. 5,494,668).

### Ashwagandha (Withania somnifera)

Ashwagandha, described as a rasayana plant in Ayurveda, possesses anti-inflammatory, antitumor, antistress, antioxidant, immunomodulatory, hemopoietic, and rejuvenating properties. Withaferin A, an important withanolide of Ashwagandha has antiarthritic and antirheumatic activity. Ashwagandha also induces NOS expression in macrophages.

This plant is native to the Indian sub-continent and has been fairly well studied for its chemistry, pharmacology and clinical efficacy. (Sharma, K. et. al., Indian Drugs, 1992, 29(6): 247-55). Ashwagandha (Withania somnifera Dunal) is widely used in Ayurvedic medicine. It is an ingredient of many formulations prescribed for a variety of musculoskeletal conditions (e.g., arthritis, rheumatism), and as a general tonic to increase energy, improve overall health and longevity. (Chatterjee, A. et al., The Treatise on Indian Medicinal Plants, 1995, 4:208-12; Bone, K., Phytotherapy Press, 1996,137-41) Many pharmacological studies have been conducted to investigate the properties of Ashwagandha in an attempt to authenticate its use as a multipurpose medical agent. Studies indicate Ashwagandha possesses anti-inflammatory, antitumor, antistress, antioxidant, immunomodulatory, hemopoietic, and rejuvenating properties. (Singh, B. et. al., Phytother Res, 2003,17(5):531-6; Bhattacharya, S. K. et. al., Pharmacol Biochem Behav, 2003, 75(3):547-55; Gupta, S.K. et. al., Drug Metabol Drug Interact, 2003,19(3):211-22; Mishra, L.C. et. al., Alternative Medicine Review, 2000, 5:334-45; Rasool, P., et. al., Vascul Pharmacol, 2006, 44(6): 406-10; Spellman, K., et. al., Altern Med Rev, 2006, 11(2):128-150). Recently, the selective Th1 up-regulating activity of withania somnifera aqueous extract has been reported (Bani, S., et. al., J Ethnopharmacol, 2006, 107(1):107-15)

The chemistry of ashwagandha has been extensively studied and over 35 chemical constituents have been identified, extracted and isolated. (Rastogi, RP. et. al., Compendium of Indian Medicinal Plants, 1998, 6). The biologically active chemical constituents are alkaloids (isopelletierine, anaferine), steroidal lactones (withaolides, withaferins), saponins containing an additional acyl group (sitoindoside VII and VIII), and withanolides with a glucose at carbon 27(sitoindoside IX and X). It is also rich in iron. Withaferin A is the most important of the withanolides. There are both animal and clinical experimental evidence for potential antiarthritic and antirheumatic activity of Ashwagandha. It has fairly potent analgesic and antiinflammatory properties.( Anabalgen, K . et. al., Indian J Exp Biol, 1981, 19: 245-49; Somasundaram, S. et. al., Clin Exp Pharmacol Physiol, 1983, 10: 147-152; Somasundaram, S. et. al., Biochem Med, 1983, 29:259-64). A bulk of evidence manifests an apparent antiinflammatory and antiarthritic activity of the plant against various models of inflammation in carageenan, cotton pellet granuloma and adjuvant arthritis where long term administration of Ashwagandha has shown significant radiographical changes.( Begum,VH. et. al., Biochem Med Metab Biol, 1987, 38: 272-77; Begum,VH. et. al., Indian J Exp Biol, 1988, 26:877-882; al Hindawi et. al., J Ethnopharmacol, 1992, 37:113-16).

In a double blind, placebo-controlled crossover study, 42 patients with osteoarthritis were randomnized to receive a formulation containing ashwagandha or placebo for three months. Patients were evaluated for one month, pretreatment, during which all previous drugs were withdrawn. During both pretreatment and treatment phase, pain and disability scores were evaluated weekly while erythrocyte sedimentation (SED) rate and radiological studies were conducted monthly. The herbal formulation significantly reduced the severity of pain (p<0.001) and disability (p<0.05) scores, although no significant changes in radiological or SED rate were noted.(Kulkarni, RR. Et al.,J Ethnopharmacol, 1991,33:91-95. Long term administration of a formulation containing Ashwagandha has shown beneficial radiological changes, reduction in erythrocyte sedimentation rate and acute phase symptoms including C-reactivity proteins. Aswagandha when given in predetermined dosages in a composition will not cause any severe side effects or undesired effects. Given alone it has limited long term benefits. (Patwardhan, U.S. Pat. No. 5,494,668).

The effect of a methanolic extract from the root of Withania somnifera (WS) on nitric oxide (NO) production in J774 macrophages was investigated. WS (1-256 µg/ml) produced a significant and concentration-dependent increase in NO production, an effect which was abolished by N(G)nitro-L-arginine methyl ester (L-NAME, 3-300 microM), a non-selective inhibitor of NO synthase (NOS), dexamethasone (10 microM), an inhibitor of protein synthesis and N(alpha-p)-tosyl-L-lysine chloromethyl ketone (TLCK, 0.01-10 microM), an inhibitor of nuclear factor-kappaB (NF-kappaB) activation. Dexamethasone did not have any effect on NO production once NOS had been induced (i.e. 12 h after WS). Moreover, a concentration-dependent increase in inducible NOS protein expression was observed. These results demonstrate that WS may induce the synthesis of inducible NOS expression likely by acting at transcriptional level. The increased NO production by macrophages could account, at least in part, for the immunostimulant properties of Withania somnifera (luvone, T. et. al., Life Sci, 2003, 72(14): 1617-25).

### Guduchi (Tinospora cordifolia)

Guduchi, described as a rasayana plant in Ayurveda is used for its immunomodulatory, adaptogenic, antistress and antioxidant properties. It inhibits prostaglandin and leukotriene biosynthesis and histamine release. Thus it acts as an anti-inflammatory as well as an antacid agent.

Guduchi is widely used in Ayurvedic medicines. It is known for its immunomodulatory, antihepatotoxic, antistress and antioxidant properties. It has been used in combination with other plant products to prepare a number of Ayurvedic preparations. (Tasaduq, SA. et. al., Hum Exp Toxicol, 2003, 22(12): 639-45; Diwanay, S. et. al., J Ethnopharmacol, 2004, 90(1): 49-55; Goel, HC. et. al., Indian J Exp Biol, 2002, 40(3): 282-7.). Guduchi, a rasayana plant from Ayurveda has been shown to have adaptogenic potential (Rege, N.N. et. al., Phytother Res, 1999,13(4): 275-91). The immunomodulatory activity of tinospora cordiflia has been widely studied (Nair, P.K. et. al., lnt Immunopharmacol,2004,4(13):1645-59; Jagetia, G.C. et.al., J Med Food, 2004,7(3):343-8; Spellman, K., et. al., Altern Med Rev, 2006, 11(2):128-150).

The active principles of Guduchi were found to possess anticomplementary and immunomodulatory activities. Syringin (TC-4) and cordiol (TC-7) inhibited the in vitro immunohaemolysis of antibody-coated sheep erythrocytes by guinea pig serum. The reduced immunohaemolysis was found to be due to inhibition of the C3-convertase of the classical complement pathway. However, higher concentrations showed constant inhibitory effects. The compounds also gave rise to significant increases in IgG antibodies in serum. Humoral and cell-mediated immunity were also dose-dependently enhanced. Macrophage activation was reported for cordioside (TC-2), cordiofolioside A (TC-5) and cordiol (TC-7) and this activation was more pronounced with increasing incubation times. (Kapil, A. et. al. J Ethnopharmacol, 1997, 58(2): 89-95).T.cordifolia, has been found to be a potent immunostimulant, with effects comparable to lithium and glucan.(Thatte, U.M. et. al., Methods Find Exp Clin Pharmacol, 1988, 10(10):639-44). Immunomodulatory and anti-tumor actions of medicinal plant Tinospora cordifolia are mediated through activation of tumor-associated macrophages ( Singh, N. et. al., lmmunopharmacol lmmunotoxicol, 2004, 26(1):145-62).

### Gokshur(Tribulus terrestris)

Tribulus terrestris has potent anti-inflammatory activity through inhibition of COX-2.

The inhibitors of prostaglandin biosynthesis and nitric oxide production have been considered as potential anti-inflammatory and cancer chemopreventive agents. Methanol extract of Tribulus terrestris has been tested for the inhibition of prostaglandin E (2) production (for COX-2 inhibitors) and nitric oxide formation (for iNOS inhibitors) in lipopolysaccharide (LPS)-induced mouse macrophages RAW264.7 cells. Tribulus terrestris showed potent inhibition of COX-2 activity (>80% inhibition at the test concentration of 10 microgram/ml) (Hong, C.H.et. al., J Ethnopharmacol, 2002, 83(1-2):153-9)

### Amla (Phyllanthus emblica)

Amla has been well studied for its hepatoprotective, antioxidant, antifungal, antimicrobial and anti-inflammatory activities.( Bandyopadhyay, S.K. et. al., J Ethnopharmacol,2000, 70(2):171-6). The anti-oxidant and immunomodulatory properties of Amla have been tested using chromium (VI) as an immunosuppressive agent. Amla relieved the immunosuppressive effects of Cr on lymphocyte proliferation and even restored the IL-2 and gamma-IFN production considerably (Sai Ram, M. et. al., J Ethnopharmacol, 2002, 81(1):5-10). Analgesic activity of extracts of Emblica officinalis fruits was evaluated. Preliminary phytochemical screening of the extracts showed the presence of alkaloids, tannins, phenolic compounds, carbohydrates and amino acids, which may be responsible for anti-pyretic and analgesic activities( Perianayagam, J. B. et. al., J Ethnopharmacol, 2004, 95(1):83-5)

The immunomodulatory properties of amla (Emblica offianalis) was evaluated in adjuvant induced arthritic rat model. Injecting Complete Freund's Adjuvant (CFA) in right hind paw of the animals induced inflammation. The crude extract of the herb was administered intraperitonially following a repeated treatment profile. The extract showed a marked reduction in inflammation and edema. At cellular level immunosuppression occurred during the early phase of the disease. There was mild synovial hyperplasia and infiltration of few mononuclear cells in treated animals. The induction of nitric oxide synthase (NOS) was significantly decreased in treated animals as compared to controls. These observations suggest that both the herbal extracts caused immunosuppression in adjuvant induced arthritic rats, indicating that it may provide an alternative approach to the treatment of arthritis (Ganju, L. et. al., Biomed Pharmacother, 2003, 57(7):296-300). An activity-directed fractionation and purification process was used to identify the nitric oxide (NO) scavenging components of Phyllanthus emblica. Five compounds showing strong NO scavenging activity were identified by spectral methods ((1)H NMR, (13)C NMR, and MS) and by comparison with literature values to be Gallic acid, Methyl gallate, Corilagin, Furosin, and Geraniin( Kumaran, A. et. al., Plant Foods Hum Nutr, 2006, 61(1):1-5;

Amla extract possesses antisecretory, antiulcer, and cytoprotective properties. Oral administration of Amla extract at doses 250 mg/kg and 500 mg/kg significantly inhibited the development of gastric lesions in all test models used. It also caused significant decrease of the pyloric-ligation induced basal gastric secretion, titratable acidity and gastric mucosal injury. Besides, Amla extract offered protection against ethanol-induced depletion of stomach wall mucus and reduction in nonprotein sulfhydryl concentration. (Al-Rehaily, A.J., Phytomedicine, 2002, 9(6): 515-22) Methanolic extract of amla was found to have ulcer protective and healing effects which might be due to its effects both on offensive (reduces acid output, pepsin output) and defensive (increases mucin secretion, cellular mucus and life span of mucosal cells) mucosal factors (Sairam K. et. al., J Ethnopharmacol, 2002, 82(1): 1-9).

### Boswellia serrata (Salai guggul)

Boswellia serrata, Linn F (Burseraceae) also known as salai guggul is commonly used in Indian system of medicine (Ayurveda) as an anti-inflammatory, analgesic, anti-arthritic and antiproliferative agent. A series of chronic inflammatory diseases are thought to be perpetuated by leukotrienes.Compounds from the gum with genuine anti-inflammatory effects are pentacyclic triterpenes of the boswellic acid type. Boswellic acids inhibit the leukotriene biosynthesis in neutrophilic granulocytes by a non-redox, noncompetitive inhibition of 5-lipoxygenase (Ammon HP. Eur J Med Res. 1996 May 24;1(8):369-70). Boswellic acids binding to the enzyme trigger the effect. Moreover certain boswellic acids have been described to inhibit elastase in leukocytes, to inhibit proliferation, induce apoptosis and to inhibit topoisomerases of leukomaand glioma cell lines. In clinical trials promising results were observed in patients with rheumatoid arthritis, chronic colitis, ulcerative colitis, Crohn's disease,bronchial asthma und peritumoral brains edemas (Ammon HP. Wien Med Wochenschr. 2002;152(15-16):373-8; Gupta I, et al. Eur J Med Res. 1997;2(1):37-43).

An open multi-centre veterinary clinical trial, comparing conditions before and after treatment with a herbal dietary supplement consisting of a natural resin extract of Boswellia serrata, was conducted on 29 dogs with manifestations of chronic joint and spinal disease Already after two weeks of treatment, an overall efficacy of the dietary supplement was evident in 71% of 24 eligible dogs. A statistically significant reduction of severity and resolution of typical clinical signs in individual animals, such as intermittent lameness, local pain and stiff gait, were reported after 6 weeks (Reichling J et al. 2004 Feb;146(2):71-9).

A randomized double blind placebo controlled crossover study was conducted to assess the efficacy, safety and tolerability of Boswellia serrata Extract (BSE) in 30 patients of osteoarthritis of knee All patients receiving drug treatment reported decrease in knee pain increased knee flexion and increased walking distance. The frequency of swelling in the knee joint was decreased (Kimmatkar N, et al. 2003 Jan;10(1):3-7).

The clinical efficacy of a herbomineral formulation containing roots of Withania somnifera, the stem of Boswellia serrata, rhizomes of Curcuma longa and a zinc complex (Articulin-F), was evaluated in a randomized, double-blind, placebo controlled, cross-over study in patients with osteoarthritis. Treatment with the herbomineral formulation produced a significant drop in severity of pain (P less than 0.001) and disability score (P less than 0.05). Side effects observed with this formulation did not necessitate withdrawal of treatment (Kulkarni RR, et al. J Ethnopharmacol. 1991 May-Jun; 33(1-2):91-5).

The effect of water-soluble fraction of the oleoresin gum of Boswellia serrata extract on lipopolysaccharide (LPS) induced nitric oxide (NO) production by macrophages under in vivo and in vitro conditions has been investigated. Under in vitro experiments with thioglycolate activated macrophages, it inhibited LPS induced (NO) production with IC 50 value at 662 ng /ml. (Pandey RS et al. Indian J Exp Biol. 2005; 43(6):509-16).

Recently, gene expression studies have been done to characterize the underlying mechanism for the anti-inflammatory effects of Boswellia extract (Roy S et al. DNA Cell Biol. 2005; 24(4): 244-55).

The herbal formulations contain therapeutically effective amounts of crude powder or hydroalcoholic extract of Shunthi and extract of Guduchi, as platform drug that can be combined with one or more of the plants Ashwagandha, Gokshur, Amalaki, Salai guggul thereby reducing/alleviating the symptoms of musculoskeletal diseases. These plants have been extensively studied. Rationale and brief review of their use in musculoskeletal diseases is provided herein.

Analgesic and anti-inflammatory activity studies for crude drug powders or extracts and formulations there off were carried out in known animal models. Central (formalin-induced model) and peripheral (acetic acid induced model) analgesic activities were studied in albino mice, while wistar rats were used for acute (carrageenan induced paw edema model) and subacute (turpentine oil-induced granuloma pouch model) anti-inflammatory activity. Suitable positive and negative controls were used for the studies. The data obtained is statistically significant (P value<0.05) and reproducible.

### Example1

### Formulation B - Shunthi-quduchi-ashwaaandha-gokshur:

The herbal formulation B contains therapeutically effective amounts of crude powder of shunthi, and aqueous extract of guduchi, ashwagandha and gokshur thereby reducing/alleviating the symptoms of musculoskeletal diseases.

The formulation is prepared by blending therapeutically effective amounts of crude ginger powder (shunthi) and extract of Guduchi stems *(Tinospora cordifolia*) to prepare a base formulation, which is further blended with therapeutically effective amounts of each of the extracts of Gokshur fruits *(Tribulus terrestris*) and Ashwagandha roots (*Withania somnifera*) sufficient to treat degenerative musculoskeletal disease. Herein each of the plant part extracts is prepared by first cleaning the plant part to remove any foreign matter; particulating the plant part to obtain a particulate mass having a particle size ranging from 2-5mm; packing the coarse powder in an extractor; charging water to the extractor for first wash; heating the material to 90-95°C; continuing the extraction for 3hours circulating the water extract from top to bottom; collecting all washes and concentrating to thick paste under vacuum; drying the thick paste in vacuum tray dryer; collecting the dried powder from vacuum tray dryer and milling in super mill; sieving in sifter using 40-mesh size. Ginger rhizomes are processed by cleaning the rhizomes to remove foreign matter; washing the rhizomes with water; drying the rhizomes in vacuum tray dryer; powdering the dried ginger in clean stainless steel grinder by using 4mm and 0.5mm mesh; sieving the powder through 40 mesh screen. The 40 mesh ginger powder is subsequently mixed with Tinospora cordifolia extract to make a thick paste for base formulation. The final formulation is prepared by blending base formulation with extracts of Gokshur (Tribulus terrestris) and Ashwagandha (Withania somnifera).

The formulation of the present invention is thus a homogenous mixture of extracts of 19.8% by weight of Gokshur fruits (*Tribulus terrestris*)*,* 26.9% by weight of Ashwagandha roots (Withania somnifera), 11.4% by weight of Guduchi stems (Tinospora cordifolia) and crude drug powder of Shunthi rhizomes (*Zingiber officinale*)*,* sufficient to treat degenerative musculoskeletal disease. Further, it is a homogenous mixture of base formulation (18% Guduchi (*Tinospora cordifolia*) water extract + 82% Shunthi (Zingiber officinale) crude drug powder) 59.92 percent, Ashwagandha (Withania somnifera) 29.47 percent and Gokshur (Tribulus terrestris) 10.61 percent of the mass of active ingredient.

### Pre-clinical Animal studies:

Formulation B and its constituent extracts of shunthi, Guduchi, ashwagandha and gokshur were found to be safe up to dose of 2000mg/kg, when given in single oral dose and monitored for 14 days as per OECD guidelines.

Experiments to evaluate analgesic and anti-inflammatory activities of extracts of guduchi, ashwagandha and gokshur were carried out. For analgesic studies doses of 10-100mg/kg-body weight were used, while for anti-inflammatory studies doses of 50-200mg/kg were used. Guduchi, gokshur and ashwagandha extract showed significant peripheral analgesic activity at 25mg/kg dose. Significant anti-inflammatory activity was observed at dose of 50mg/kg of ashwagandha and 100mg/kg of gokshur and guduchi in acute model of inflammation. Significant sub-acute anti-inflammatory activity was observed at dose of 50mg/kg of ashwagandha, gokshur and guduchi extract in exudative model. The formulation B showed significant dose reduction for peripheral analgesic activity and acute inflammatory activity. Significant peripheral analgesic activity and acute anti-inflammatory activity was observed at 25mg/kg dose of the formulation. Significant sub-acute anti-inflammatory activity was observed at dose of 400mg/kg of the formulation in exudative model.

### Cell line studies

The formulation B as well as crude powders and extracts of the individual herbs used in the formulation were studied for protection against nitric oxide damage in human OA chondrocytes. Combination of shunthi, guduchi, ashwagandha and gokshur in formulation B showed significant protection against NO damage in human OA chondrocytes.

| **Crude Drug Powder or Extract** | | | |
|---|---|---|---|
| | **Model** | | |
| **Test Materials** | **Protection against NO damage on Human OA chondrocytes** | **Protection against peroxide damage** | **Improvement in general chondrocyte viability** |
| Shunthi crude powder | NA | NA | |
| Guduchi water extract | 30-200% (N=3) | 26.33% ±4.5% (n=2) | 30.4% ±6% (N=3) |
| Gokshur water extract | NA | NA | |
| Ashwagandha extract water | NA | 39.6%±14% (n=3) | 25.33% ±0.5% (n=2) |

| **Formulation** | | | |
|---|---|---|---|
| | | **Decrease in PG release** | |
| Formulation B | 40.00%±10.98% (n=4) | IC50=50mg/ml(n=3) | |

### Clinical Study:

The said formulation was evaluated in clinical trials on patients with osteoarthritis and
rheumatoid arthritis. The details of the trials are given below:
   NMITLI OA stage I/2: The said formulation was evaluated in a randomized double blind placebo controlled, multicentric 16 week clinical trial in 245 patients with symptomatic knee osteoarthritis. This was This was a 7 arm exploratory study aimed to evaluate the efficacy and
   safety of 5 multiplant formulations using Shunthi-Guduchi as a common platform formulation in all. Glucosamine was the positive comparator. There were no significant differences between the arms but efficacy trends favored the said formulation in particular comparison to glucosamine. The formulation was as safe as placebo.

### NMITLI OA STAGE I/4:

A randomized double blind parallel efficacy 16-week study conducted in 108 to evaluate the efficacy and safety of the said formulation as compared to two proprietary ayurvedic formulations. Overall, the efficacy results were modest with no significant difference between the 3 arms.

### NMITLI OA STAGE I/5:

Twenty-eight patients still symptomatic with knee OA after completing NMITLI OA stage I/2 study and willing to continue were followed up in an open label study of 16 weeks duration. All patients received the said formulation and the majority was able to sustain fair efficacy with minimal mild side effects.

### NMITLI OA STAGE I/6:

Forty-nine patients completing NMITLI OA STAGE I/4 with active pain VAS ≥4 were continued in an open label study. They were randomized into two arms- formulation B and a proprietary ayurvedic formulation. Formulation B demonstrated a fair efficacy response with minimal side effect profile.

NMITLI RA STAGE I/1: A randomized double blind comparator controlled parallel efficacy multicentric 24-week study in patients with active Rheumatoid arthritis (RA). In this study, formulation B and a proprietary ayurvedic formulation were compared to Hydroxychloroquine (HCQS), which is a popular DMARD (Disease Modifying Anti Rheumatoid Drug) in modern medicine rheumatology. As a complex painful inflammatory polyarticular disease, several efficacy variables, both clinical and laboratory were used to evaluate efficacy- Joint Counts for pain/tenderness and swelling, pain VAS, Patients Global assessments by the patient and physician, Health Assessment Questionnaire (a validated instrument for functional ability for Indian use), ESR, CRP and cytokines markers.

At baseline, all the groups were matched well in several demographic and efficacy measures. There were no significant differences between the treatment arms. However, in individual group analysis, there were several significant improvements in both formulation 'B' and HCQS and both showed almost similar results at week 24. The overall safety of Ayurvedic formulations was better than HCQS.

To the best of our knowledge, this probably is the first time in the World that a long-term study has demonstrated an equally effective but better safety profile for an Ayurvedic formulation (in this case formulation B) as compared to a modern medicine DMARD in a difficult to treat disease such as RA.

NMITLI RA STAGE I/2: Patients completing NMITLI RA STAGE I/1 and willing to continue were further followed up in a double blind cross-over trial design of 24 weeks duration in CRD. Patients were crossed between formuation B and the proprietary ayurvedic formulation. Patients completing HCQS intervention were randomized into either of the two Ayurvedic arms (Formulation B and proprietary ayurvedic formulation). Throughout this study, the patient and the trial doctor were blinded to the treatment arm. The data of this trial has been entered and the statistical analysis is awaited.

### Analgesic activity:

1. Crude powder or extract:

| Test material | Minimum Dose Showing Significant Activity (Mg/Kg) | |
|---|---|---|
| | Central | Peripheral |
| Shunthi crude powder | NA | 100 |
| Guduchi water extract | NA | 25 |
| Ashwagandha water extract | NA | 25 |
| Gokshur water extract | NA | 25 |

2. Formulation:

| Test material | Minimum Dose Showing Significant Activity (Mg/Kg) | |
|---|---|---|
| | Central | Peripheral |
| Shunthi+ Guduchi+ Ashwagandha+Gokshur | NA | 25 |

### Anti-inflammatory activity:

1. Crude powder or extract:

| Test material | Minimum Dose Showing Significant Activity (Mg/Kg) | |
|---|---|---|
| | Acute | Sub-acute |
| Shunthi crude powder | 400 | 400 |
| Guduchi water extract | 100 | 50 |
| Ashwagandha water extract | 50 | 50 |
| Gokshur water extract | 100 | 50 |

2. Formulation:

| Test material | Minimum Dose Showing Significant Activity (Mg/Kg) | |
|---|---|---|
| | Acute | Sub-acute |
| Shunthi+ Guduchi + Ashwagandha+Gokshur | 25 | 400 |

### Example 2

### Shunthi-guduchi-amalaki (Formulation C):

The herbal formulation C contains therapeutically effective amounts of hydroalcoholic extract of shunthi, and aqueous extract of guduchi, amalaki and salai guggul thereby reducing/alleviating the symptoms of musculoskeletal diseases.

The formulation is prepared by first cleaning the plant part to remove any foreign matter; particulating the plant part to obtain a particulate mass having a particle size ranging from 2-5mm; packing the coarse powder in an extractor; charging solvent (soft water used for guduchi and amalaki; 90 percent ethanol used for shunthi ) to the extractor for first wash; heating the material (90°C for guduchi and amalaki; 70°C for shunthi) continuing the extraction for 3hours circulating the water extract from top to bottom; collecting all washes and concentrating to thick paste under vacuum; drying the thick paste in vacuum tray dryer; collecting the dried powder from vacuum tray dryer and milling in super mill; sieving in sifter using 40-mesh size. The final formulation is prepared by blending all three extracts in desired proportion.

### Pre-clinical Study:

### Animal Studies:

### 1. Genotoxicity studies:

Assessment of genotoxicity using mouse bone marrow micronucleus test and Salmonella reverse mutation test was carried out according to OECD guidelines No.474 and 471 respectively.

In bone marrow micronucleus test, there was no increase in percent micronuclei indicating that the formulation possesses no mutagenic effect when tested in mammalian system. The results of Salmonella reverse mutation test suggest that the formulation is not a direct acting mutagen nor a pro-mutagen when tested for strains TA97, TA98, TA100 and TA102.

Acute oral toxicity studies suggest that the said formulation is safe up to 2000mg/kg dose when given in single dose and monitored for 14 days as per OECD guidelines.

The formulation shows significant peripheral analgesic activity and acute and subacute anti-inflammatory activity in animal models of pain and inflammation.

### 2. Cell line studies:

**The formulation shows 20.8% +/-7% (n=3) inhibition of hyaluronidase enzyme (400U/ml) activity at 0.60mg/ml. It also shows 30%+/-11% (n=4) protection against NO damage in human**

| **Crude Drug Powder or Extract** | | | |
|---|---|---|---|
| | **Model** | | |
| **Test Materials** | **Protection against NO damage on Human OA chondrocytes** | **Protection against peroxide damage** | **Inhibition of *hyaluronidase*** |
| Shunthi crude powder | **NA** | **NA** | |
| Guduchi water extract | 30-200% (N=3) | 26.33% ±4.5% (n=2) | |
| Amalaki extract | 50% ± 4% (n=3) | | IC50=0.30mg/ml (n=3) |

| Formulation | | | |
|---|---|---|---|
| | | **Decrease in PG release** | |
| Formulation C | 30% ± 11% (n=4) | 70% ± 5%(n=4) | 20.8% 7% (n=3) |

| | | | |
|---|---|---|---|
| OA cartilage. | | | |

### Clinical Study:

The formulation shows better efficacy than glucosamine in a randomized double blind, placebo & comparator controlled multicentric 16-week study in patients with symptomatic knee

Osteoarthritis (OA). Glucosamine was the positive comparator in this study. The formulation was found to be as safe as placebo.

**1. Analgesic activity:**

| **Extract** | | |
|---|---|---|
| Test material | Minimum Dose Showing Significant Activity (Mg/Kg) | |
| | Central | Peripheral |
| Shunthi Hydroalcoholic extract | NA | NA |
| Guduchi water extract | NA | 25 |
| Amalaki water extract | NA | 25 |

| Formulation | | |
|---|---|---|
| Shunthi- Guduchi -Amalaki | NA | 400 |

**2. Anti-inflammatory activity:**

| **Extract** | | |
|---|---|---|
| Test material | Minimum Dose Showing Signficant Activity (Mg/Kg) | |
| | Acute | Sub-acute |
| Shunthi Hydroalcoholic extract | 100 | NA |
| Guduchi water extract | 100 | 50 |
| Amalaki water extract | 50 | 50 |

| **Formulation** | | |
|---|---|---|
| Shunthi- Guduchi -Amalaki | NA | NA |

### Example 3

### Formulation C+G - (shunthi+ auduchi+ amalaki + guggul):

Shunthi (Zingiber officinale) hydro-alcoholic extract was standardized with respect to 6-gingerol, 8-gingerol, 10-gingerol, 6-shogaol and total gingerols.

Salai guggul (Boswellia serrata)hydroalcoholic extract was standardized with respect to total boswellic acid percentage, boswellic acid (alpha+beta), alpha-boswellic acid, beta- boswellic acid, alpha-acetyl-boswellic acid, beta-acetyl- boswellic acid, 11-keto-beta -boswellic acid, acetyl-11-keto-beta- boswellic acid and acetyl-boswellic acid (alpha+beta).

Amla (Phyllanthus emblica)extract was standardized with respect to total tannin as tannic acid and gallic acid.

Guduchi (Tinospora cordifolia) extract was standardized with respect to marker tinosporoside.

### Preclinical Animal studies -

### a. Acute oral safety studies:

Formulations C and C+G were found to be safe up to the dose of 2000 mg/kg when given single oral dose and monitored for 14 days as per OECD (Organisation for Economic Cooperation and Development) guidelines No. 423. No symptoms related to acute toxicity were observed.

### b. Genotoxicity Study:

Genotoxicity study was carried out using mammalian bone marrow micronucleus test with the limit test at a single dose level of 2000 mg/kg body weight. The test drug was suspended in 1 % CMC (carboxy methyl cellulose) and administered orally to the experimental animals. Control animals received 1% CMC alone. A positive control group treated with cyclophosphamide at the dose of 75 mg/kg by i.p. route was also employed to demonstrate the sensitivity of the test.

Treatment was given for two consecutive days at 0 h and 24 h. The treated mice were observed for toxic signs/symptoms and mortality during the study period. All the mice were sacrificed at 6^{th} hr. after the last treatment (30^{th} hr. after first dose) and femur bones were excised and bone marrow contents were flushed with fetal calf serum. Bone marrow cells were centrifuged spread as a smear on glass slide and stained using Giemsa stain and May-Grunwald stain. The slides were observed for polychromatic erythrocytes (PCEs), normochromatic erythrocytes (NCEs) and micronucleated PCEs. Minimum 2000 PCEs were scored from each animal. All the slides were coded prior to scoring and decoded after completion of scoring. No clinical signs were observed in any of the treated as well as control group animals. All the animals were found to be normal prior to sacrifice. No treatment related alterations were observed in the treated group of Formulation C and C+G. The micronucleus induction in control and treated groups were comparable as observe in % micronuclei per PCE.

| **Crude Drug Powder or Extract** | | | |
|---|---|---|---|
| | **Model** | | |
| **Test Materials** | **Protection against NO damage on Human OA chondrocytes** | **Protection against peroxide damage** | **Inhibition of hyluronidase** |
| Shunthi crude powder | NA | NA | |
| Guduchi water extract | 30-200% (N=3) | 26.33%±4.5% (n=2) | |
| Amalaki extract | 50% ± 4% (n=3) | | IC50=0.30mg/ml (n=3) |
| Salai guggul extract | | Inhibition of collagenase II-MIC-5mg.ml (n=3) | IC50=4mg.ml (n=3) |

| Formulation | | | |
|---|---|---|---|
| | Decrease | **Decrease in PG release** | |
| Formulation C+G | 30-70% (n=3) | 81.68+ 22.76 (n=3) | 33.58 + 10.60% (n=3) |

Micronucleus induction in positive control group (Cyclophosphamide treated) was significantly high when compared with the control group, which demonstrate the sensitivity of the test. The results were analyzed using Student's t test. The positive control cyclophosphamide showed significant increase in percent micronuclei: PCE ratio, thereby confirming its mutagenic effect. However, both formulations did not produce micronuclei in the immature erythrocytes and possesses no mutagenic effect when tested in mammalian system.

### c. Pharmacological studies (analgesic and anti-inflammatory activity):

Analgesic activity of formulations C and C+G was determined by evaluation of the anti-nociceptive response of the test samples. The following models were used for these studies:
a) Acetic acid induced writhing in mice (peripheral pain model)
   The total number of writhings following intra-peritoneal administration of 0.6% acetic acid, 10 ml / Kg body weight was recorded for 20 minutes, starting 10 minutes after injection. The animals were pre-treated with test samples (100, 200 and 400mg/Kg p.o.) 30 minutes prior to above treatment, simultaneously using Aspirin (30mg/kg p.o.) as a positive control.
b) Tail flick method in mice (central analgesic model)
   The tail flick response was tested 30 minutes after treatment of test drug sample (100, 200 and 400mg/Kg p.o.) and Pentazocine (25mg/kg P.O) as a positive control. The latency period in seconds taken by the animal for tail flick response when its tail is immersed in a water bath maintained at constant temperature of 52-55⁰C was recorded in various groups.
c) Formalin induced paw licking in mice (model for differentiation of the mechanism) - This method is based on the ability of the drug to inhibit the nociceptive responses (licking and biting) by a mouse after injection of 0.05ml formalin (1%) in the plantar aspects of the hind limb. Summation of time in seconds spent in licking and biting of formalin injected paw during each 5-min block is measured for 30 min as an indicator of pain response. Duration of responses at 0 to 5 min and that from 20-30 min represent central and peripheral mechanism of action of drug under consideration respectively. This test was performed 30 minutes after treatment of test sample (100, 200 and 400 mg/Kg p.o.) and Aspirin (200 mg/kg p.o.) as a standard and the time (sec.) taken by the animal to show the nociceptive response was recorded.

**1. Analgesic activity:**

| **Extracts** | | |
|---|---|---|
| Test material | Minimum Dose Showing Significant Activity (Mg/Kg) | |
| | Central | Peripheral |
| Shunthi Hydroalcoholic extract | NA | NA |
| Guduchi water extract | NA | 25 |
| Amalaki water extract | NA | 25 |
| Salai guggul ethanolic extract | NA | 400 |

| **Formulation** | | |
|---|---|---|
| Shunthi- Guduchi -Amalaki-Salai guggul | NA | 100 |

**2. Anti-inflammatory activity:**

| **Extract** | | |
|---|---|---|
| Test material | Minimum Dose Showing Significant Activity (Mg/Kg) | |
| | Acute | Sub-acute |
| Shunthi Hydroalcoholic extract | 100 | NA |
| Guduchi water extract | 100 | 50 |
| Amalaki water extract | 50 | 50 |
| Salai guggul ethanolic extract | 400 | NA |

| **Formulation** | | |
|---|---|---|
| Shunthi- Guduchi -Amalaki-Salai guggul | NA | 400 |

| | | |
|---|---|---|
| NA= no activity when tested up to 400mg/kg dose of the test material | | |

### Example 4

### Acute and sub-acute anti-inflammatory studies were carried out using following models:

1. Carrageenan induced rat paw edema -
   Various doses of test sample (100, 200 and 400mg/Kg p.o.) and standard Indomethacin (10mg/kg p.o.) were administered to different groups of rats. Acute inflammation was induced half an hour after the above treatment by injection of 0.1 ml of freshly prepared 1% (in water) suspension of Carrageenan sodium in sub-planter region of rat right hind paw. Paw volume was measured by plethysmographic method initially and then at 1, 2, 3, 4, 24, 48 and 72 hrs after carrageenan injection.
2. Turpentine oil-induced granuloma pouch in rats
   Sub-cutaneous dorsal granuloma pouch was prepared in rats by injecting air (2ml), followed by injection of 0.5 ml of turpentine oil in it. Test sample at various doses (100, 200 and 400mg/Kg p.o.) and Indomethacin (1mg/kg p.o.) was administered to different groups of rats for 7 consecutive days. The pouch was opened on 7^{th} day under ether anesthesia and exudate was taken out by syringe and the volume of exudate was compared in various groups. The Total Leukocyte Count from exudative fluid was determined by Neubaurer's chamber using micropipette.

### Results:

The results of studies on analgesic and anti-inflammatory activity are summarized below:
1. Formulation C+G showed significant peripheral analgesic activity and sub-acute anti-inflammatory activity at 100mg/kg dose.
2. No central analgesic activity was observed at higher dose of 400mg/kg for standard anti-arthritic drugs glucosamine and celecoxib while peripheral analgesic activity was observed in acetic acid induced writhing for both the drugs at minimum dose of 400mg/kg. The results of the anti-inflammatory studies show presence of significant anti-inflammatory activity for celecoxib at minimum dose 200mg/kg while glucosamine was devoid of any anti-inflammatory activity.

Formulation C+G is more potent than celecoxib with respect to analgesic and anti-inflammatory activity. Compared to glucosamine, it is pore potent with respect to analgesic activity and has added advantage of anti-inflammatory activity. Thus Formulation C+G can be beneficial both in treatment and management of arthritis.

### Example 5

### In vitro studies:

Cytoprotection in human chondrocytes, prevention of cartilage matrix degradation and inhibition of the enzyme hyaluronidase were the principle approaches used during the study. The chondroprotective and anti-inflammatory activities of formulations Cand C+G were examined.

Standard anti-arthritic drugs Glucosamine and celecoxib were used as positive controls. Four *in vitro* assays were used to evaluate the chondroprotective potential of formulations C and C+G.

These included assays for Glycosaminoglycan (GAG), aggrecan and nitric oxide (NO) release from cartilage explant from chronic OA patients undergoing knee replacement surgery and assay for inhibition of Hyaluronidase activity. The methodology and results are given below:

### Methods:

### Preparation of Formulation C and C+G extracts:

Aqueous extracts of each capsule were freshly prepared as required. Contents of each capsule were solubilized in distilled water (10mG/mL) by limited autoclaving. Each extract was filter sterilized prior to addtion to cartilage explants at the required final concentration. This method of herbal extract preparation gave reproducible sterility and biological activity (Sumantran et al, 2007).

### Osteoarthritis Patient profile:

Patients (age 55-75 years) suffered from chronic osteoarthritis (OA) for 5-15 years prior to knee replacement surgery. Cartilage integrity was recorded by macroscopic and microscopic examination, using the Outerbridge scale. Only non-calcified, grade 1 - 2 articular cartilage from the lateral femoral condyles was used (Outerbridge 1961).

### Explant cultures of OA cartilage:

OA cartilage explants were cultured as described earlier (Sumantran et al, 2007). Briefly, explants were treated for 24 hours with/without sterile aqueous extracts of each capsule, and re-fed with growth media without herbal extract every two days for eight days. Conditioned media (CM) samples from each explant were collected prior to each re-feed. All CM samples per OA patient were thawed and assayed for GAG and NO levels simultaneously. Aggrecan assays were done after analysis of the GAG release data.

### Assays for cartilage explant damage:

### glycosaminoglycans (GAG), and Nitric oxide (NO) assay

Total GAG levels in CM samples were measured by the dimethylmethylene blue assay using Chondroitin sulphate as a standard (Hoemann et al, 2002). NO levels in CM samples were estimated using the Griess reagent.

### Aggrecan assay

Selected CM samples from cartilage explants which showed a significant decrease in GAG release in response to a specific formulation were quantitated for aggrecan using an Enzyme amplified immunoassay (EAISA) kit (Biosource, Belgium). The kit uses a mixture of monoclonal antibodies to different epitopes of aggrecan, thereby ensuring sensitive and rapid estimation of aggrecan.

### Data calculation:

GAG content is expressed as microgram equivalents of Chondroitin sulphate. NO is expressed in micromoles / mG of explant / mL of CM. Units of Aggrecan are micrograms/ mG of explant /mL of CM. Each marker is measured at each time point for each CM sample derived from each patient.

### Statistical Analysis of glycosaminoglycan, aggrecan and Nitric oxide data :

Levels of GAG, NO, or aggrecan levels in CM samples from explants at each time point, per patient, treated with/without drug; were tested for statistical significance at the 95% confidence level using the Students 2 tailed t-test for paired samples. We calculated the effect of drug extracts in each CM sample, at each time point, per patient sample; using the ratio shown below:

### GAG, NO, or aggrecan levels in CM from drug treated explants

GAG, NO, or aggrecan levels in CM from control explants.

Long- term versus Short- term activity: Long- term chondroprotective or anti-inflammatory activity is the ability of a drug to cause a statistically significant decrease in levels of GAG, aggrecan, or NO release by OA cartilage explants, relative to controls. This effect must be observed for at least 3 of the 4 time points during in vitro culture. Short- term activity is similar, but is detected at only 1 or 2 of the 4 time points tested.

### Results:

### P. emblica aqueous extract

Aqueous extracts of *P.emblica* fruit and Glucosamine sulphate showed comparable chondroprotective activity in explant model of OA cartilage damage. In addition, *P*. *emblica* extract strongly inhibited Hyaluronidase activity, and moderately inhibited the gelatinase activity of type 2 collagenase in vitro.

### T. cordifolia stem aqueous extract

Aqueous extracts of *T.cordifolia* stem (0.05 mG/mL) caused a statistically significant 40.67 + 14.33% increase in viability of human OA chondrocytes in culture (n=3 patients). This extract also protected the chondrocytes from NO damage induced by the NO donor, Sodium Nitroprusside (SNP). Thus, OA chondrocytes pre-treated with *T.cordifolia* extract had 30.40 ± 6.50% greater viability than chondrocytes treated with SNP alone (n=4 patients). These results suggest that extracts of *T*.*cordifolia* stem can improve viability of human OA chondrocytes, and provide a rationale for its inclusion in formulations C and C+G.

### B. serrata and Z. officinale

Effects of hydro-alcoholic extracts of these 2 herbs on OA cartilage damage could not be evaluated, since the alcohol solvent itself caused cartilage damage. However, these herbs were included in the formulations due to reports of their beneficial role in clinical trials for joint disease (Altman et al 2001; Kimmatkar et al 2003).

### Example 6

### Chondroprotective activities of formulation C and C+G:

### Glyrcosaminoglycan (GAG) release from human OA cartilage explants:

In explant model of OA cartilage damage, GAG released by cartilage explants for 5 time points after a 24 hour drug treatment was measured. The effect on GAG release from cartilage explants of six OA patients treated with/without aqueous extracts of formulations C and C+G (100microg/mLeach)was studied. Data analyses showed that Formulation C caused a statistically significant decrease in GAG release at time points 3 and 4, relative to corresponding controls. Although the magnitude of chondroprotection by formulation C was small, (20-23% reduction in GAG release relative to controls), it was detected at two successive time points. Formulation C+G caused a statistically significant 22%-30% decrease in GAG release at three time points (points 2,3 and 4), compared to corresponding controls.

Glucosamine and celecoxib each caused a statistically significant, 25% decrease in GAG release at time point 4, compared to controls.

In summary, Formulation C, glucosamine and celecoxib each exhibited significant short-term chondroprotection at time points 3 and/or 4 in cartilage explants from six OA patients. Formulation C+G caused significant long-term chondroprotection in explants from these same patients. Interestingly, both formulations and positive controls caused chondroprotection at time point 4.

### Example 7

### Dose dependence of chondroprotective activities of the formulations:

The two formulations and positive controls exhibit dose dependent effects (Table1).

Formulations C and C+G gave chondroprotective activity at 100 microg/mL only, whereas glucosamine and celecoxib show chondroprotective activity at 50 and 100 microg/mL. A possible reason for differences in the effective doses of the four s relates to their composition.

Formulations C and C+G are a mixture of herbal extracts, whereas, glucosamine and celecoxib are pure compounds.

### Aggrecan release from human OA cartilage explants:

Data in Table 1 showed that FormulationC+G induced significant long-term chondroprotective effects on OA cartilage from 6 patients. Therefore, we assayed for aggrecan levels in CM samples of the explants from these six OA patients treated with/without Formulation C+G (100microg/mL), at time points 3 and 4. Since Glucosamine reportedly inhibits aggrecanase (Uittertinden et al, 2006), CM samples from Glucosamine treated explants from these patients were included as positive controls.

### Example 8

### Effects of Formulation C+G and Glucosamine on Glycosaminoglycan and Aggrecan

### release by OA cartilage:

Figures 1 and 2 show comparative effects of Formulation C+G and glucosamine on the release of total GAG and aggrecan by cartilage explants from the six OA patients analysed in Table 1. Data for time points 3 and 4 are shown in Figures 1 and Figure 2 respectively.
Figure 1A shows that Formulation C+G induced a mean, statistically significant, 33-38% decrease in levels of GAG and aggrecan release, relative to levels of these markers released by control explants from the six patients at time point 3. Glucosamine also induced a mean, statistically significant, 25-35% decrease in levels of GAG and Aggrecan release, by explants from five of these six patients, relative to levels of these markers released by controls (Figure 1B). When we compare efficacy of Formulation C+G and glucosamine in each patient sample at time point 3; we observe that both were equally effective for reduction of release of both markers by explants from patients 3 and 5. However, glucosamine was not as effective as formulation C+G in reducing GAG and aggrecan release by cartilage explants from patients 1,2, and 4.
Figure 2A shows that Formulation C+G induced a mean, statistically significant, 32-40% decrease in levels of GAG and Aggrecan release by explants from the six patients relative to levels of these markers released by controls at time point 4. Figure 2B shows effects of glucosamine on cartilage damage in five of these six cases. On close examination, glucosamine was as effective as Formulation C+G in decreasing release of both markers by explants from patients 1 and 5. Both formulation C+G and glucosamine effectively reduced GAG release by explants from patients 2 and 4 relative to GAG release by controls. However, at time point 4, aggrecan release by explants from patients 2 and 4 were significantly decreased by Formulation C+G, but not glucosamine.

To summarize, Formulation C+G effectively decreased release of GAG and aggrecan from OA cartilage at time points 3 (Figure 1A) and 4 (Figure 2A). In contrast, glucosamine significantly reduced release of these markers at time point 3 only (Figure 1B). Based on these results, formulation C+G was a more effective chondroprotective agent than Glucosamine, the positive control in these six OA patients. Furthermore, the magnitude of reduction of aggrecan release caused by Formulation C+G and glucosamine (Figures 1 and 2) is sufficient to account for the observed reduction of GAG release by cartilage explants from these patients (Table 1). Therefore, the primary mechanism of chondroprotection for Formulation C+G and 3 involves significant inhibition of aggrecan loss by OA cartilage in these patients.

### Example 9

### Formulation C+G transiently reduces Nitric Oxide release by OA cartilage explants:

The effects of each both formulations on levels of NO secreted by OA cartilage explants was tested. Formulation C+G (100microg/mL) treated explants released 64.38 +_17.02% of NO levels released by controls (100%). Interestingly, this reduction of NO release by Formulation C+G was statisticaly significant, and coincided with the chondroprotective effects of Formulation C+G on explant cultures from the six OA patients at time point 4. Formulation C, gulosamine and celecoxib did not significantly decrease NO release by explants from these six patients at any time point. These data demonstrate a unique anti-inflammatory potential for Formulation C+G.

### Example 10

### Comparison of anti-arthritic properties of Formulation C and C+G

As shown above, Formulation C+G exhibited dual chondroprotective and anti-inflammatory properties in our model of OA cartilage damage. In contrast, Formulation C did not significantly decrease aggrecan or NO release by cartilage explants from these six OA patients. Since oleoresin of *B. serrata* is the only ingredient which is present in Formulation C+G, but not in FormulationC; we suggest that *B.serrata* synergises with a component (s) of Formulation C (T. *cordifolia* stem, fruit of *P*. *emblica,* and/or *Z*. *offcinale* root), to give the unique anti-arthritic properties of Formulation C+G observed in vitro. When we consider properties of the individual herbal components of Formulation C and Formulation C+G, we note that *P.emblica* fruit significantly inhibited Hyaluronidase Activity (IC50 for Hyaluronidase inhibition=0.30mG/mL, manuscript in press). However, Formulation C and C+G weakly inhibited Hyaluronidase (IC 50 values = 2.0-5.0 mG/mL). Similarly, each component of Formulation C and Formulation C+G moderately inhibited the gelatinase activity of type 2 collagenase; whereas Formulation C and Formulation C+G lacked collagenase inhibitory activity. These data suggest that the chondroprotective activity of Formulation C+G is independent of the Hyaluronidase and collagenase inhibitory activities of its individual herbal components.

Rather, the ability of Formulation C+G to significantly inhibit aggrecan, and NO release from OA cartilage explants, maybe the crucial mechanism underlying its unique chondroprotective activity.

Table 2 compares the anti-arthritic activities of the four formulations. Formulation C+G has the triple property of significantly decreasing release of Glycosaminoglycan, aggrecan, and NO, from OA cartilage explants. In contrast, Formulation C (the parent formulation for Formulation C+G), only inhibits GAG release transiently. As reported in the literature, glucosamine, significantly decreased release of GAG and aggrecan from OA cartilage. Our data on celecoxib ( Tables 1 and 2), is consistent with a report showing that celecoxib enhanced proteoglycan content without altering NO levels in early and end-stage OA cartilage (Mastbergen et al, 2005).

**Table 1**

| **Time point** | **Percent control GAG release** | **Percent control GAG release** | **Percent control GAG release** | **Percent control GAG release** |
|---|---|---|---|---|
| | **Formulation C** | **Formulation C+G** | **Glucosamine** | **Celecoxib** |
| 1 | 88.38 ± 44.88 | 95.39 ± 38.61 | 118.96 ± 34.74 | 90.24 ± 25.71 |
| 2 | 94.36 ± 23.10 | ***78.85* ± *23.00*** | 91.70 ± 16.55 | 84.87 ± 28.54 |
| 3 | *81.77* ± *17.23* | ***67.82* ± *16.45*** | 86.52 ± 19.36 | 87.83 ± 28.99 |
| 4 | *77.48* ± *17.92* | ***71.14* ± *20.68*** | *76.01* ± *19.96* | *74.81* ± 31.83 |
| 5 | 99.73 ± 34.73 | 79.92 ± 25.16 | 87.15 ± 20.83 | 89.42 ± 31.98 |

### Table 1: Summary of Chondroprotective activity of formulations

Glycosaminoglycan (GAG) release from cartilage explants from OA patients treated with/without aqueous extracts of each of the four capsules (100microg/mL) is shown. Each number represents GAG release from cartilage explants of OA patients treated with a particular capsule (Mean and Standard Deviation), expressed as a percentage of levels of GAG released by corresponding control explants. GAG release from control explants is set at 100%. Numbers in italics indicate a value which was statistically significantly lower than control. Formulation C, C+G and celecoxib, caused short-term chondroprotection. Formulation C+G caused long-term chondroprotection (in bold italics). For Formulation C, C+G and celecoxib samples from six patients were used. For Glucosamine, samples from five of these six OA patients were used.

**Table 2**

| **Test Formulation** | **Glycosaminoglyan (GAG) release** | **Aggrecan (AGG) release** | **Nitric oxide (NO) release** |
|---|---|---|---|
| **Formulation C** | Short term decrease | No significant decrease | No significant decrease |
| **Formulation C+G** | Long term decrease | Significant decrease | Significant decrease |
| **Glucosamine** | Short term decrease | Significant decrease | No significant decrease |
| **Celecoxib** | Short term decrease | Not tested | No significant decrease |

### Table 2: Comparison of Anti-arthritic activities of the four formulations

Formulation C+G significantly decreased release of glycosaminoglycan, aggrecan, and NO, from OA cartilage explants. Formulation C, only caused short-term inhibition of cartilage damage (GAG release). Glucosamine significantly decreased release of GAG and aggrecan from OA cartilage. Celecoxib, only caused short term inhibition of cartilage damage. Only Formulation C+G caused a significant transient decrease in levels of NO released by OA cartilage.

### Example 11

The clinical trials are conducted on the following basis:
◆ Randomized, Double Blind, parallel efficacy, Placebo & Comparator controlled study
◆ 245 patients in a 7 arm (5 ayurvedic arms coded as A-E, placebo-1, Glucosamine-1) study of 16 weeks duration.
◆ 35 patients in each arm
◆ Monitored pain rescue medication (oral paracetamol) permitted on SOS basis
◆ Intent to treat analysis with last observation carried forward
◆ All arms well-matched at baseline for demographics and efficacy. Best efficacy trends shown by 'B' and 'C' formulations which were numerically superior to Placebo & Glucosamine in several efficacy measures (statistically not significant)
◆ Only minor adverse events were recorded in each of the arms.

**Table 3**

| Drug Cod | PainVAS (0-100mm) | WOMAC Pain (0-20 score) | PATG (1-5 grades) |
|---|---|---|---|
| B | 60.3, 28% | 8.3, 11.6% | 3.5, 17.7% |
| C | 60.3, 28% | 7.9, 26.7% | 3.49, 20.9% |
| GLS | 64.8, 21% | 8.0, 14.2% | 3.52, 15.4% |

Note: PainVAS- VISUAL ANALOGUE SCALE- Maximum pain on active weight bearing activity during last 24 hours. (0- 100 mm)a horizontal scale, anchored at 0 for no pain and 100 for maximum possible pain.

WOMAC- a questionnaire based instrument to measure functional ability or disability due to disorders of knee and hip joint A modified version validated for Indian use was used (www.rheumatologyindia.org)

PATG- Patient global assessement of overall disease recorded in 5 grades-Asymptomatic, Mild, Moderate, Severe, Very Severe; B, C- Ayurvedic formulations; GLS- Glucosamine

**Table 4**

| Knee status change on completion - A RIDIT (relative to identified distribution) analysis | | | |
|---|---|---|---|
| COMPARISON PAIR | MEAN RIDIT | Z VALUE | P-VALUE |
| C Vs Placebo | 0.652 | 2.185 | 0.029* |
| C Vs Glucosamine | 0.618 | 1.692 | 0.091 Marginally significant |

Interpretation (for mean Ridit >0.5) - More than half of the time a randomly selected subject from 'C' group will have more extreme value (improvement) than a randomly selected subject from the reference group (Placebo & Glucosamine)

**Table 5**

| | | N | Mean | Sd. | Minimum | Maximum | F |
|---|---|---|---|---|---|---|---|
| AVR_PAR3 | A | 29 | 2.2438 | 1.0781 | .14 | 4.29 | 2.5* |
| | B | 29 | 1.3645 | 1.1020 | .00 | 3.71 | |
| | C | 29 | 1.3227 | 1.2704 | .00 | 4.29 | |
| | D | 30 | 1.3762 | 1.1305 | .00 | 4.29 | |
| | E | 30 | 1.7429 | 1.0270 | .00 | 3.57 | |
| | Gls | 26 | 2.0110 | 1.5801 | .00 | 4.29 | |
| | Plb | 29 | 1.6675 | 1.1658 | .00 | 4.21 | |
| | Total | 202 | 1.6694 | 1.2245 | .00 | 4.29 | |
| AVR_PAR4 | A | 29 | 2.3621 | 1.0091 | .36 | 4.29 | 1.81 |
| | B | 29 | 1.6108 | 1.3595 | .00 | 4.29 | |
| | C | 29 | 1.5690 | 1.3810 | .00 | 4.29 | |
| | D | 30 | 1.4595 | 1.1729 | .00 | 4.29 | |
| | E | 30 | 2.0262 | 1.2580 | .00 | 4.07 | |
| | Gls | 26 | 1.9038 | 1.5246 | .00 | 4.29 | |
| | Plb | 29 | 1.6675 | 1.1488 | .00 | 4.29 | |
| | Total | 202 | 1.7977 | 1.2853 | .00 | 4.29 | |
| AVR_PAR5 | A | 29 | 2.4951 | 1.2261 | .00 | 4.29 | 1.58 |
| | B | 29 | 1.9544 | 1.4303 | .00 | 4.29 | |
| | C | 29 | 1.7734 | 1.5246 | .00 | 4.29 | |
| | D | 30 | 1.5107 | 1.3689 | .00 | 4.07 | |
| | E | 30 | 2.2726 | 1.2790 | .00 | 4.29 | |
| | Gls | 26 | 1.9863 | 1.5287 | .00 | 4.29 | |
| | Plb | 29 | 1.8350 | 1.4498 | .00 | 4.29 | |
| | Total | 202 | 1.9744 | 1.4147 | .00 | 4.29 | |
| AVR_PAR6 | A | 29 | 2.0333 | 1.0450 | .00 | 3.57 | 2.45* |
| | B | 29 | 1.7586 | 1.0586 | .00 | 3.21 | |
| | C | 29 | 1.1761 | 1.2435 | .00 | 3.21 | |
| | D | 30 | 1.3071 | .9455 | .00 | 2.93 | |
| | E | 30 | 1.8488 | 1.2133 | .00 | 4.64 | |
| | Gls | 26 | 1.8846 | 1.1914 | .00 | 3.21 | |
| | Plb | 29 | 1.4335 | 1.1344 | .00 | 3.21 | |
| | Total | 202 | 1.6303 | 1.1459 | .00 | 4.64 | |
| AVR_PAR7 | A | 29 | 1.4889 | .5985 | .00 | 2.14 | 2.32* |
| | B | 29 | 1.2500 | .7759 | .00 | 2.93 | |
| | C | 29 | .9076 | .7557 | .00 | 2.14 | |
| | D | 30 | 1.0167 | .8540 | .00 | 2.14 | |
| | E | 30 | 1.4655 | .8014 | .00 | 2.82 | |
| | Gls | 26 | 1.3516 | .7988 | .00 | 2.14 | |
| | Plb | 29 | 1.1835 | .8355 | .00 | 2.86 | |
| | Total | 202 | 1.2360 | .7936 | .00 | 2.93 | |
| AVR_TOTAL | A | 29 | 2.0800 | .8103 | .34 | 3.39 | 2.59* |
| | B | 29 | 1.6127 | .9216 | .00 | 3.19 | |
| | C | 29 | 1.3257 | 1.0540 | .02 | 3.30 | |
| | D | 30 | 1.3131 | .9663 | .00 | 2.68 | |
| | E | 30 | 1.8679 | .8837 | .00 | 3.16 | |
| | Gls | 26 | 1.7950 | 1.1201 | .01 | 3.36 | |
| | Plb | 29 | 1.5299 | .9225 | .11 | 3.13 | |
| | Total | 202 | 1.6436 | .9784 | .00 | 3.39 | |

A-E, ayurvedic groups; Plb- Placebo, Gls-Glucosamine; PAR-Paracetamol
i)Except at visit 4 and 5 a small significant difference at p<0.05 was seen at all visits {Significant F (ANOVA) =2.17}
ii) Minimum paracetamol consumption was observed in Groups D and C (for'C' significant low
   consumption at visit 6 and visit 7)

### Example 12

### DOSING STUDY

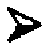 Single blind study of 6 weeks duration meant to evaluate the maximum tolerable dose
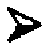 Sample size of 86 patients
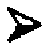 4 best ayurvedic formulations (B, B+, C+. D+) from earlier exploratory studies
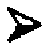 No rescue pain medication was permitted
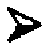 All groups matched well at Baseline for demography & efficacy measures
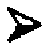 No statistically significant difference between arms for all efficacy measures
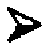 The C+ group formulation showed the maximum improvement in reducing pain (painVAS) and WOMAC pain and was numerically superior to all other groups
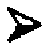 No significant adverse events were recorded

**Table 6 - Baseline mean & mean percent change over 6 weeks**

| | PainVAS (0-100mm) | WOMAC Pain (0-20 score) | PATG (1-5 grades) |
|---|---|---|---|
| B | 63.0, 12.5% | 7.9, 7.3% | 3.5, 4.05% |
| B+ | 65.0, 10.2% | 7.6, -1.2% | 3.7, 12.1% |
| C+ | 65.0, 21.5% | 8.9, 15.8% | 3.8, 12.1% |
| D+ | 62.0, 8.5% | 7.6, -3.4% | 3.5, 12.5% |

### Example 13

### PHASE II STUDIES:

∘ Phase II, Randomized, double blind, comparator (Celecoxib & Glucosamine) controlled, multicentric trial of equivalence.
∘ Study Duration- 24 weeks
∘ Sample size (statistically powered 80% with α <0.05) - 440 patients with symptomatic OA knees
∘ 4 arm- 2 Ayurvedic Interventions (C, C+) Celecoxib & Glucosamine, parallel efficacy study
∘ 110 patients in each arm
∘ No rescue medication permitted

### PHASE II STUDY EXTENDED:

87 Patients from above cohort completed 48 weeks of treatment in 1 center, with sustained efficacy & good safety profile

**Table 7 - Mean change between baseline and completion**

| **Variable** | **Drug code** | | | | **P** |
|---|---|---|---|---|---|
| | **OA-01** | **OA-02** | **OA-03** | **OA-04** | |
| | **Mean ± SD** | **Mean ± SD** | **Mean ± SD** | **Mean ± SD** | |
| PainVAS | -2.04 ± 2.20 | -2.45 ± 2.23 | -2.06 ± 2.42 | -1.82 ± 1.95 | 0.21 |
| W_PAIN | -2.26 ± 3.29 | -2.72 ± 3.26 | -1.78 ± 3.35 | -1.89 ± 1.95 | 0.14 |
| W_STIFF | -0.40 ± 1.71 | -0.49 ± 1.61 | -0.30 ± 1.43 | -0.34 ± 1.91 | 0.86 |
| W_DIFF | -6.74 ± 10.71 | -8.12 ± 10.89 | -5.85 ± 9.18 | -6.93 ± 9.88 | 0.45 |
| W_TOTAL | -9.42 ± 14.04 | -11.33 ± 13.75 | -7.94 ± 12.64 | -9.17 ± 13.05 | 0.32 |

### Codes used:

| **Code** | **Formulation** |
|---|---|
| **A** | Shunthi+ Guduchi+ Gokshur |
| **B** | Shunthi+ Guduchi+ Ashwagandha+ Gokshur |
| **C** | Shunthi+Guduchi+ Amalaki |
| **D** | Shunthi+ Guduchi |
| **E** | Shunthi+ Guduchi+ Ashwagandha |
| **OA-01** | Shunthi+ Guduchi+ Amalaki+ Salai Guggul |
| **OA-02** | Glucosamine |
| **OA-03** | Shunthi+ Guduchi+ Amalaki |
| **OA-04** | Celecoxib |

### Advantages of the invention:

The advantages of the present invention are:
1. Herbal in nature and hence has no side effects
2. The formulation provides long term protection against arthritis.

## Claims

1. A synergistic herbal composition for use in the treatment of rheumatic and musculo-skeletal disorders (RMSDs) comprising a base formulation (50-60%) and plant parts and/or extracts of the plant (40-50%) selected from the group consisting of *Withania somnifera, Tribulus terrestris, Phyllanthus emblica* and *Boswellia serrata* and any combination thereof optionally along with pharmaceutically acceptable excipients, wherein the base formulation essentially consists of *Zingiber officinale* and *Tinospora cordifolia* in a ratio in the range of 10:90 - 20:80.

2. A synergistic formulation for use according to claim 1, wherein the said formulation preferably comprising *Zingiber officinale, Tinospora cordifolia, Withania somnifera* and *Tribulus terrestris* in a ratio in the range of 20:40:20:20-30:20:25:25 forming formulation B having peripheral analgesic activity and acute inflammatory activity.

3. A synergistic formulation for use according to claim 1, wherein the said formulation preferably comprising *Zingiber officinale, Tinospora cordifolia* and *Phyllanthus emblica* in a ratio in the range of 1:4:8 - 3:5:12 forming formulation C having peripheral analgesic activity and acute inflammatory activity; or
wherein the said formulation preferably comprising *Zingiber officinale, Tinospora cordifolia* and *Boswellia serrata* in a ratio in the range of 1:4:5 - 3:5:7 forming formulation G having peripheral analgesic activity and acute inflammatory activity.

4. A synergistic herbal formulation for use according to claim 1 , wherein the said formulation further preferably comprising *Zingiber officinale, Tinospora cordifolia, Phyllanthus emblica* and *Boswellia serrata* in a ratio in the range of 1:4:8:5 - 3:5:12:7 forming formulation (C+G).

5. A synergistic herbal formulation for use according to claim 1, wherein the said formulation is capable of causing simultaneous long term decrease in glycosaminoglycan (GAG) release and aggrecan by cartilage explants resulting in reduction of pain, inflammation, stiffness and low degeneration of bones, joints, muscles and other connective tissues; or
wherein the said formulation is non toxic at least up to a single dose of 2000mg/Kg body weight of the subject in need.

6. A synergistic herbal formulation for use according to claim 1, wherein the therapeutically effective dose of the said formulation is in the range of 20-300 mg/kg of the subject in need.

7. A synergistic herbal formulation for use according to claim 6, wherein the said formulation is capable of being used as an analgesic in an effective dose in the range of 10-100mg/Kg of body weight of the subject in need; or
wherein the said formulation is capable of being used as an anti-inflammatory agent in an effective dose in the range of 50-400mg/Kg of body weight of the subject in need; or wherein the said formulation is having acute and sub-acute inflammatory activity at an effective dose in the range of 25-400mg/Kg of body weight of the subject in need; or wherein the said formulation is capable of being used as a sub-acute anti-inflammatory agent in an effective dose in the range of 20-300 mg/kg of body weight of the subject in need.

8. A synergistic herbal formulation for use according to claim 1, wherein the said formulation is capable of protecting against Nitric oxide damage in human osteo-arthritic chondrocytes; or
wherein the said formulation is useful for Symptomatic treatment of Osteoarthritis (OA) up to 12 months of therapy; or
wherein the said formulation is capable of protecting against peroxide damage.

9. A synergistic herbal formulation for use according to claim 2, wherein the said formulation B is inhibiting hyaluronidase enzyme activity up to 20.8 ± 7%.

10. A synergistic herbal formulation for use according to claim 4, wherein the said formulation (C+G) is inhibiting hyaluronidase enzyme activity up to 33.58 ± 10.6%; or
wherein the formulation (C+G) is at least up to 25-40% more effective than glucosamine for reducing the glycosaminoglycan (GAG) and aggrecan release.

11. A synergistic formulation for use according to claim 1, wherein the said formulation is in the form selected from the group comprising powder, capsule, tablet, liquid, paste.

12. A process for the preparation of a synergistic herbal composition as claimed in claim 1, wherein the said process comprising the following steps of:
a. grinding the plant materials to obtain particle size ranging between 2 - 5 mm for obtaining powdered plant material;
b. optionally packing the coarse powder in an extractor followed by water-extraction method for obtaining an plant extract;
c. charging water into the powder as obtained from step (a) followed by heating at a temperature range of 90 - 95 degree C for a period of at least up to 3 hours;
d. concentrating the thick paste as obtained from step (c) under vacuum followed by drying using a drier then milling and sieving thereof to obtain the desired formulation of dried plant powder or plant extract.

13. A process as claimed in claim 12, wherein the plant materials are selected from the group comprising a base formulation of *Zingiber officinale* and *Tinospora cordifolia,* and plant parts and/or extract of plants selected from the group consisting of *Withania somnifera, Tribulus terrestris, Phyllanthus emblica, Boswellia serrata*; or
wherein the formulation B comprising rhizome of *Zingiber officinale,* stem of *Tinospora cordifolia,* roots of *Withania somnifera* and fruits of *Tribulus terrestris*; or
wherein the plant part used is selected from the group comprising stem, bark, root, flower bud and fruit.

14. A process as claimed in claim 12, wherein the *Withania somnifera* used being in the range of 25-30% by weight of the said herbal formulation; or
wherein the *Tribulus terrestris* used being in the range of 10-20% by weight of the said herbal formulation; or
wherein the excipient used is preferably starch; or
wherein the sieving is performed preferably in a sifter using 30-50µm mesh size.

## Patentansprüche

1. Eine synergistische pflanzliche Zusammensetzung zur Verwendung der Behandlung von rheumatischen oder muskuloskelettalen Erkrankungen (RMSDs) umfassend eine Basisformulierung (50-60%) und Pflanzenteile und/oder Extrakte der Pflanze (40-50%) ausgewählt aus der Gruppe bestehend aus *Withania somnifera, Tribulus terrestris, Phyllanthus emblica* und *Boswellia serrata* und irgendeine Kombination davon, optional gemeinsam mit pharmazeutisch akzeptablen Trägern, wobei die Basisformulierung im Wesentlichen aus *Zingiber officinale* und *Tinospora cordifolia* in einem Verhältnis im Bereich von 10:90-20:80 besteht.

2. Eine synergistische Formulierung zur Verwendung nach Anspruch 1, wobei diese Formulierung vorzugsweise *Zingiber officinale, Tinospora cordifolia, Withania somnifera* und *Tribulus terrestris* in einem Verhältnis im Bereich von 20:40:20:20-30:20:25:25 umfasst, die Formulierung "B", mit peripherer analgetischer Wirkung und akuter inflammatorischer Wirkung, bildet.

3. Eine synergistische Formulierung zur Verwendung nach Anspruch 1, wobei diese Formulierung vorzugsweise *Zingiber officinale, Tinospora cordifolia* und *Phyllanthus emblica* in einem Verhältnis im Bereich von 1:4:8-3:5:12 umfasst, die Formulierung C mit peripherer analgetischer Wirkung und akuter inflammatorischer Wirkung bildet; oder
wobei diese Formulierung vorzugsweise *Zingiber officinale, Tinospora cordifolia* und *Boswellia serrata* in einem Verhältnis im Bereich von 1:4:5-3:5:7 umfasst, die Formulierung G, mit peripherer analgetischer Wirkung und akuter inflammatorischer Wirkung bildet.

4. Eine synergistische pflanzliche Formulierung zur Verwendung nach Anspruch 1, wobei diese Formulierung weiter bevorzugt *Zingiber officinale, Tinospora cordifolia, Phyllanthus emblica* und *Boswellia serrata* in einem Verhältnis im Bereich von 1:4:8:5-3:5:12:7, die Formulierung (C+G) bildet, umfasst.

5. Eine synergistische pflanzliche Formulierung zur Verwendung nach Anspruch 1, wobei diese Formulierung in der Lage ist, eine simultane Langzeitverringerung in der Glykosaminoglykan (GAG) Freisetzung und Aggrecan durch Sehnenexplantate zu bewirken, wodurch Schmerz, Entzündungen, Steifigkeit und geringe Degeneration von Knochen, Gelenken, Muskeln und anderen Bindegewebe reduziert wird; oder wobei diese Formulierung bis mindestens zu einer Einzeldosis von 2000 mg/Kg Körpergewicht des betroffenen Subjekts nicht toxisch ist.

6. Eine synergistische pflanzliche Formulierung zur Verwendung nach Anspruch 1, wobei die therapeutische effektive Dosis dieser Formulierung im Bereich von 20-300 mg/Kg des betroffenen Subjekts liegt.

7. Eine synergistische pflanzliche Formulierung zur Verwendung nach Anspruch 6, wobei diese Formulierung geeignet ist, als Analgetikum in einer effektiven Dosis im Bereich von 10-100 mg/Kg des Körpergewichts des betroffenen Subjekts eingesetzt zu werden; oder
wobei diese Formulierung geeignet ist, als ein anti-inflammatorisches Agens in einer effektiven Dosis im Bereich von 50-400 mg/Kg Körpergewicht des betroffenen Subjekts eingesetzt zu werden; oder
wobei diese Formulierung eine akute und sub-akute inflammatorische Wirkung bei einer effektiven Dosis im Bereich von 25-400 mg/Kg Körpergewicht des betroffenen Subjekts aufweist; oder
wobei diese Formulierung geeignet ist, als ein sub-akutes anti-inflammatorisches Agens in einer effektiven Dosis im Bereich von 20-300 mg/Kg Körpergewicht des betroffenen Subjekts eingesetzt zu werden.

8. Eine synergistische pflanzliche Formulierung zur Verwendung nach Anspruch 1, wobei diese Formulierung geeignet ist, Schutz gegen Stickoxidschaden in menschlichen osteo-arthritischen Chondrozyten zu verleihen; oder
wobei diese Formulierung zur symptomatischen Behandlung von Osteoarthritis (OA) bis zu 12 Monaten Therapie verwendbar ist; oder
wobei diese Formulierung geeignet ist, gegen Peroxid-Schaden zu schützen.

9. Eine synergistische pflanzliche Formulierung zur Verwendung nach Anspruch 2, wobei diese Formulierung B die Hyaluronidase-Enzymaktivität bis zu 20,8 ± 7% inhibiert.

10. Eine synergistische pflanzliche Formulierung zur Verwendung nach Anspruch 4, wobei diese Formulierung (C+G) die Hyaluronidase-Enzymaktivität bis zu 33,58 ± 10,6% inhibiert; oder
wobei die Formulierung (C+G) in der Reduzierung der Freisetzung von Glykosaminoglykan (GAG) und Aggrecan mindestens bis zu 25-40% effektiver als Glukosamin ist.

11. Eine synergistische Formulierung zur Verwendung nach Anspruch 1, wobei diese Formulierung in der Form ausgewählt aus der Gruppe umfassend Pulver, Kapsel, Tablette, Flüssigkeit und Paste bereitgestellt wird.

12. Ein Verfahren zur Herstellung einer synergistischen pflanzlichen Zusammensetzung wie in Anspruch 1 beansprucht,
wobei dieses Verfahren folgenden Schritt umfasst:
a) Zermahlen des Pflanzenmaterials um Partikelgrößen im Bereich zwischen 2 und 5 mm zu erhalten, um pulverförmiges Pflanzenmaterial zu erhalten;
b) optional Beschicken eines Extraktors mit dem Rohpulver gefolgt von einem Wasserextraktionsverfahren zum Erhalt eines Pflanzenextrakts;
c) Hinzufügen von Wasser in das Pulver, wie in Schritt (a) erhalten, gefolgt durch Erwärmen in einem Temperaturbereich von 90-95°C für eine Dauer von mindestens bis zu 3 Stunden;
d) Konzentrieren der dicken Paste, erhalten aus Schritt (c), unter Vakuum, gefolgt von einer Trocknung unter Verwendung eines Trockners mit nachfolgendem Zermahlen und Sieben desselben zum Erhalt der gewünschten Formulierung des getrockneten Pflanzenpulvers oder Pflanzenextrakts.

13. Ein Verfahren wie in Anspruch 12 beansprucht,
wobei die Pflanzenmaterialien ausgewählt sind aus der Gruppe, umfassend eine Basisformulierung von *Zingiber officinale und Tinospora cardifolia,* und Pflanzenteile und/oder Extrakte von Pflanzen, ausgewählt aus der Gruppe, bestehend aus *Withania somnifera, Tribulus terrestris, Phyllanthus emblica, Boswellia serrata;* oder
wobei die Formulierung B das Rhizom von *Zingiber officinale,* den Stamm von *Tinospora cordifolia,* die Wurzeln von *Withania somnifera* und die Früchte von *Tribulus terrestris* umfasst; oder
wobei der verwendete Pflanzenteil ausgewählt ist aus der Gruppe, umfassend Stamm, Borke, Wurzel, Blütenstand und Frucht.

14. Ein Verfahren wie in Anspruch 12 beansprucht,
wobei *Withania somnifera* im Bereich von 25-30 Gewichts-% der besagten pflanzlichen Formulierung eingesetzt wird; oder
wobei *Tribulus terrestris* im Bereich von 10-20 Gewichts-% der besagten pflanzlichen Formulierung eingesetzt wird; oder
wobei als Träger bevorzugt Stärke eingesetzt wird; oder wobei das Sieben bevorzugt in einem Sieb ("Sifter") unter Verwendung von 30-50 µm Maschenweite ausgeführt wird.

## Revendications

1. Composition synergique à base de plantes pour son utilisation dans le traitement des troubles rhumatismaux et musculosquelettiques (TRMS) comprenant une formulation de base (50 à 60 %) et des parties de plantes et/ou des extraits de plantes (40 à 50 %) choisies dans le groupe consistant en *Whitania somnifera, Tribulus terrestris, Phyllanthus emblica* et *Boswellia serrata* et de l'une quelconque de leurs combinaisons accompagnés éventuellement d'excipients pharmaceutiquement acceptables, dans laquelle la formulation de base consiste essentiellement en *Zingiber officinale* et *Tinospora cordifolia* dans un ratio situé dans la plage de 10/90 à 20/80.

2. Formulation synergique pour son utilisation selon la revendication 1, ladite formulation comprenant de préférence *Zingiber officinale, Tinospora cordifolia, Whitania somnifera* et *Tribulus terrestris* dans un ratio situé dans la plage de 20/40/20/20 à 30/20/25/25 formant une formulation B dotée d'une activité antalgique périphérique et d'une activité inflammatoire aiguë.

3. Formulation synergique pour son utilisation selon la revendication 1, ladite formulation comprenant de préférence *Zingiber officinale, Tinospora cordifolia* et *Phyllanthus emblica* dans un ratio situé dans la plage de 1/4/8 à 3/5/12 formant une formulation C dotée d'une activité antalgique périphérique et d'une activité inflammatoire aiguë ; ou
ladite formulation comprenant de préférence *Zingiber officinale, Tinospora cordifolia* et *Boswellia serrata* dans un ratio situé dans la plage de 1/4/5 à 3/5/7 formant une formulation G dotée d'une activité antalgique périphérique et d'une activité inflammatoire aiguë.

4. Formulation synergique à base de plantes pour son utilisation selon la revendication 1, ladite formulation comprenant en outre de préférence *Zingiber officinale, Tinospora cordifolia, Phyllanthus emblica* et *Boswellia serrata* dans un ratio situé dans la plage de 1/4/8/5 à 3/5/12/7 formant une formulation (C+G).

5. Formulation synergique à base de plantes pour son utilisation selon la revendication 1, ladite formulation étant capable de provoquer une diminution simultanée à long terme de la libération de glycosaminoglycane (GAG) et d'aggrécane par des explants de cartilage produisant une réduction de la douleur, de l'inflammation, de la raideur et une faible dégénérescence des os, des articulations, des muscles et d'autres tissus conjonctifs ; ou
ladite formulation n'étant pas toxique au moins jusqu'à une dose unique de 2000 mg/kg de poids corporel du sujet en ayant besoin.

6. Formulation synergique à base de plantes, pour son utilisation selon la revendication 1, la dose thérapeutiquement efficace de ladite formulation se situant dans la plage de 20 à 300 mg/kg du sujet en ayant besoin.

7. Formulation synergique à base de plantes, pour son utilisation selon la revendication 6, ladite formulation pouvant être utilisée en tant qu'antalgique dans une dose efficace située dans la plage de 10 à 100 mg/kg du poids corporel du sujet en ayant besoin ; ou
ladite formulation pouvant être utilisée en tant qu'agent anti-inflammatoire dans une dose efficace située dans la plage de 50 à 400 mg/kg du poids corporel du sujet en ayant besoin ; ou
ladite formulation étant dotée d'une activité inflammatoire aiguë et subaiguë à une dose efficace située dans la plage de 25 à 400 mg/kg du poids corporel du sujet en ayant besoin ; ou
ladite formulation pouvant être utilisée en tant qu'agent anti-inflammatoire subaigu dans une dose efficace située dans la plage de 20 à 300 mg/kg du poids corporel du sujet en ayant besoin.

8. Formulation synergique à base de plantes, pour son utilisation selon la revendication 1, ladite formulation pouvant protéger contre les lésions dues à l'oxyde nitrique dans les chondrocytes ostéoarthritiques humains ; ou
ladite formulation étant utile pour le traitement symptomatique de l'ostéoarthrite (OA) jusqu'à 12 mois de traitement ; ou
ladite formulation pouvant protéger contre les lésions dues aux peroxydes.

9. Formulation synergique à base de plantes telle pour son utilisation selon la revendication 2, ladite formulation B inhibant l'activité de l'enzyme hyaluronidase jusqu'à 20,8 ± 7 %.

10. Formulation synergique à base de plantes pour son utilisation selon la revendication 4, ladite formulation (C+G) inhibant l'activité de l'enzyme hyaluronidase jusqu'à 33,58 ± 10,6 % ; ou
la formulation (C+G) étant au moins jusqu'à 25 à 40 % plus efficace que la glucosamine pour la réduction de la libération de glycosaminoglycane (GAG) et d'aggrécane.

11. Formulation synergique pour son utilisation selon la revendication 1, ladite formulation étant sous une forme choisie dans le groupe comprenant une poudre, une capsule, un comprimé, un liquide, une pâte.

12. Procédé de préparation d'une composition synergique à base de plantes telle que revendiquée dans la revendication 1, ledit procédé comprenant les étapes suivantes :
a. le broyage des matériaux végétaux pour obtenir une taille de particule située dans la plage de 2 à 5 mm pour obtenir un matériau végétal en poudre ;
b. le tassement éventuel de la poudre grossière dans un extracteur suivi d'un procédé d'extraction à l'eau pour obtenir un extrait végétal ;
c. le chargement d'eau dans la poudre telle qu'obtenue à partir de l'étape (a) suivi d'un chauffage dans une plage de températures de 90 à 95 degrés C pendant une période d'au moins jusqu'à 3 heures :
d. la concentration de la pâte épaisse telle qu'obtenue à partir de l'étape (c) sous vide suivie d'un séchage en utilisant un séchoir puis d'un broyage et d'un tamisage de celle-ci pour obtenir la formulation souhaitée de la poudre de plante séchée ou de l'extrait de plante souhaité.

13. Procédé tel que revendiqué dans la revendication 12, dans lequel les matériaux végétaux sont choisis dans le groupe comprenant une formulation de base de *Zingiber officinale* et *Tinospora cordifolia,* et des parties de plantes et/ou un extrait de plantes choisies dans le groupe consistant en *Whitania somnifera, Tribulus terrestris, Phyllanthus emblica* et *Boswellia serrata* ; ou
dans lequel la formulation B comprend le rhizome de *Zingiber officinale,* la tige de *Tinospora cordifolia,* les racines de *Whitania somnifera* et les fruits de *Tribulus terrestris ;* ou
dans lequel la partie de plante utilisée est choisie dans le groupe constitué de la tige, l'écorce, la racine, le bourgeon floral et le fruit.

14. Procédé tel que revendiqué dans la revendication 12, dans lequel la *Whitania somnifera* utilisée se situe dans la plage de 25 à 30 % en poids de ladite formulation à base de plantes ; ou
dans lequel le *Tribulus terres tris* utilisé se situe dans la plage de 10 à 20 % en poids de ladite formulation à base de plantes ; ou
dans lequel l'excipient utilisé est de préférence l'amidon ; ou
dans lequel le tamisage est effectué de préférence dans un tamis en utilisant une taille de maille de 30 à 50 µm.
